# EUROPEAN PATENT APPLICATION

(11) **EP 3 640 324 A1**
(43) Date of publication of application: **22.04.2020**
(21) Application number: 18200408.5
(22) Date of filing: 15.10.2018
(51) Int. Cl.: C12N 5/071

(54) **CERVICAL CANCER ORGANOIDS**

(71) Applicant: Max-Planck-Gesellschaft zur Förderung der Wissenschaften e.V., 80539 München (DE)
(72) Inventor: GURUMURTHY, Rajendra Kumar, 10115 Berlin (DE); CHUMDURI, Cindrilla, 10115 Berlin (DE); MEYER, Thomas F., 14612 Falkensee (DE)
(74) Representative: Weickmann & Weickmann PartmbB

(57) **Abstract**

The present invention relates to a method for the production of a cervix epithelial cell organoid culture. By means of this method, an organoid culture of cervix epithelial cells, and a biobank comprising a plurality of different organoid cultures thereof may be generated. Further, a culture medium suitable for the long-term culture of epithelial stem cells is provided. Furthermore, the use of the organoid culture in the biobank for medical applications, e.g. in the field of diagnostics and therapy and in the fields of drug screening and immunotherapy is described.

## Description

The present invention relates to a method for the production of a cervix epithelial cell organoid culture. By means of this method, an organoid culture of cervix epithelial cells, and a biobank comprising a plurality of different organoid cultures thereof may be generated. Further, a culture medium suitable for the long-term culture of epithelial stem cells is provided. Furthermore, the use of the organoid culture in the biobank for medical applications is described, e.g. for the field of diagnostics and therapy and the fields of drug screening and immunotherapy, e.g. neo-epitope detection for personalized immune intervention. The invention further relates to the use of vitamin A and its derivatives for the prevention or treatment of cervical disorders including cervical cancer, precancer, and metaplasia development as well as related pathologies affecting the epithelial transitions zones in other organs.

Recent years have seen rapid progress in our understanding of how adult epithelial tissues are maintained by dedicated stem cell niches. In many cases, Wnt signaling initiated from the underlying stroma plays decisive roles. What is entirely unclear so far is how these niches are restrained at the boundary between two different types of epithelia, i.e. columnar and stratified epithelia. Respective transition zones are found in several organs in the human body, e.g. the gastro-esophageal junction, as well as the cervix. These sites almost invariably show a predisposition towards transformation, which is preceded by metaplasia, where one epithelium type invades another.

Cervical cancer predominantly occurs at the transition zone (TZ), where the stratified squamous epithelium of the ectocervix and the simple columnar epithelium of the endocervix meet (1, 2). It is one of the most common and deadly cancers in women and occurs as two histologically distinct types: adenocarcinomas (ADC) and squamous cell carcinomas (SCC) (3). Both ADC and SCC are proposed to originate from a common precursor stem cell population with a unique immunophenotype that is localized exclusively at the TZ. This precursor cell population is proposed to consist either of residual embryonic cytokeratin 7 (KRT7) positive cells (4), p63+/KRT7+/KRT5+ transitional basal cells (5), or embryonic reserve cells (6).

As a result, prophylactic ablation of residual embryonic cytokeratin 7 (KRT7) positive cells has been proposed as a method to prevent cervical cancers (37, 38). How this population gives rise to two epithelial types and the molecular mechanisms that govern maintenance of the TZ and define the distinct epithelial compartments has not been studied. In addition, the changes that contribute to metaplasia at the TZ of the cervix (or indeed other tissues) also remain unclear.

The present inventors employed *in vivo* lineage tracing, cervical organoid models, single molecule RNA *in situ* hybridization (RNA-ISH) and a mouse model of squamous metaplasia, we show that endo- and ectocervical epithelia are derived from two separate lineage-specific stem cells that meet at the TZ. Using bioinformatics analysis as well as immunohistochemistry of cancer tissue, we find that the transcriptional signatures of these lineages correspond to those of SCC and ADC, indicating that these two histologically distinct cancer types also arise from the two different lineages rather than from a common precursor. We show that two distinct active or inhibitory Wnt signaling milieus are established in the endo- and ectocervical region by Wntpathway agonists and antagonists, respectively, which are differentially expressed in the epithelium and sub-epithelial stroma on either side of the TZ *in vivo.* Organoids derived from these two distinct stem cell lineages show a strong divergence in their requirement for Wnt signaling. Strikingly, we demonstrate that the endocervix also harbors squamous stem cells, which are kept in a quiescent state by the Wnt signaling microenvironment that promotes proliferation of the columnar lineage. *In vitro,* these quiescent stem cells can give rise to squamous epithelium in the absence of Wnt. This is the first time that the presence of two stem cell lineages with opposing signaling requirements within the same niche has been observed. In addition, using an *in vivo* mouse model of cervical metaplasia, we show that loss of Wnt signals by induction of Wnt inhibitory signaling molecules in the endocervical region leads to the outgrowth of the pre-existing ectocervical stem cells within the transition zone and the endocervix. These findings provide the first mechanistic underpinning of how homeostasis is maintained at the transition zone and development of metaplasia.

Thus, a first aspect of the invention relates to a method for the production of a cervix epithelial cell organoid culture, said method comprising the steps:
(a) cultivating cervix stem cells in a suitable cell culture medium, under conditions wherein an organoid is formed, and
(b) optionally obtaining an organoid from the cultivation step (a).

As a starting material for the culture, stem cells from the cervix, particularly from the endocervix, or from the ectocervix may be used. These stem cells may be obtained from samples of dissociated cells, from cervical tissues or cells from biopsies of healthy cervix tissue. The stem cells may be derived from any mammal species, e.g. human or other primates or rodents such as mouse or rat. More particularly, cervix stem cells derived from human subjects, e.g. human patients, are used as a starting material.

In endocervical tissue, two discrete types of stem cells are present. Depending on the culture conditions endocervical or ectocervical organoids may be obtained. In ectocervical tissue, a single type of stem cells is present from which ectocervical organoids may be obtained.

The cultivation is carried out in a cell culture medium which is capable of supporting growth and/or propagation of ectocervical or endocervical organoids as described below. Any cell culture system may be used which are supportive of maintenance of stem cells such as 3D cultures and feeder based cultures.

A further aspect of the present invention is a cervix epithelial cell organoid culture which can be stably propagated, e.g. a squamous stratified ectocervical epithelial organoid cell culture or a columnar endocervical epithelial organoid cell culture. This culture is defined by the presence of squamous stratified ectocervical epithelial cells or columnar endocervical epithelial cells in the form of organoids which may have an average size of about 100-500 µm. The respective organoids were found to exhibit a characteristic morphology and/or to express characteristic cellular markers.

Ectocervical cell culture organoids, e.g. human or mouse ectocervical organoids have a morphology recapitulating the *in vivo* tissue architecture characterized by the presence of stratified epithelial layers and by the presence of the cellular marker E-cadherin. The outer layer of the organoids may consist of basal cells which are positive for the cellular marker p63. These cells may differentiate into para-basal cells with reduced levels of p63 and characteristic p63 negative cells facing the lumen. Only basal cells are positive for the proliferation marker Ki67.

Ectocervical stem cells including stem cells from ectocervical organoid cultures were found to have a high upregulation of the Wnt antagonists Dickkopf Wnt signaling pathway inhibitor 1 (DKK1), DKK3 and KREMEN2 (DKK receptor). Further, ectocervical organoids were found to be negative for the Wnt target gene LGR5. Further, human ectocervical stem cells were found to have high expression levels of the Notch ligand, Δ-like ligand 3 (DLL3) and manic fringe (MFNG). Additional upregulated genes of ectocervical stem cells are described in the Example section.

Endocervical cell culture organoids were found to consist of a large lumen with a simple columnar epithelium characterized by the presence of the cellular marker E-cadherin. A sporadic distribution of Ki67 positive proliferating cells was found. In endocervical organoids, Wnt-related genes are upregulated while Wnt antagonists and Notch-related genes are upregulated in ectocervical organoids.

Further, both stem cells and differentiated cells of ectocervical squamous organoids show high expression of the cellular marker KRT5 whereas this marker is absent from endocervical columnar organoids. In contrast, endocervical organoids show high expression of KRT7 and KRT8.

Endocervical stem cells or ectocervical stem cells may be cultivated into a respective organoid cell culture using appropriate culture media as disclosed herein.

The organoid culture is a stable culture, i.e. it can be propagated for a time of at least six months or longer in a suitable cell culture medium as described herein. The organoid culture may be subjected to freezing/thawing procedures, and expansion, e.g. into a multi-well format, suitable for high throughput, set-ups and drug testing. If desired, cells from the organoid culture may be subjected to genetic modification, e.g., by genome editing.

A further aspect of the invention is a biobank comprising a plurality of different organoid cultures. The biobank may comprise the organoid cultures in the form of individual propagating organoid cultures and/or frozen organoid cultures.

A still further aspect of the invention relates to the use of a cell culture medium for the production of a cervix epithelial cell organoid cell culture, e.g. a squamous stratified ectocervical epithelial organoid cell culture or a columnar endocervical epithelial organoid culture.

The cell culture medium may be selected from any suitable eukaryotic cell culture medium comprising salts, vitamins and trace elements. The cell culture medium for each organoid culture has specific niche requirements.

For the cultivation of murine ectocervical organoids a preferred culture medium comprises epidermal growth factor (EGF), e.g. in an amount of 5-100 ng/ml such as about 30 ng/ml, nicotinamide and an inhibitor of tissue growth factor β (TGF-β) signaling, e.g. TGF-β R kinase inhibitor IV, noggin, e.g. about 100 ng/ml mouse noggin, and a ROCK inhibitor such as Y-27632, e.g. in an amount of about 10 µM. The presence or absence of Wnt pathway activators such as Wnt3a and/or RSPO1, was found not to substantially effect the murine organoid culture.

For the cultivation of human ectocervical organoids a Wnt deficient medium is used, i.e. a medium without exogenously added activators of Wnt signaling such as Wnt3a and/or RSPO1. Further, the medium preferably comprises an activator of cAMP signaling such as forskolin. For example, a suitable medium may comprise EGF, e.g. in an amount of 1-50 ng/ml such as about 10 ng/ml, fibroblast growth factor 10 (FGF-10), e.g. in an amount of 20-250 ng/ml such as about 100 ng/ml, noggin, e.g. in an amount of 20-250 ng/ml, such as about 100 ng/ml, forskolin, a ROCK inhibitor such as Y-27632, e.g. in an amount of about 10 µM, and an inhibitor or TGF-β signaling, e.g. TGF-β R kinase inhibitor IV.

For the cultivation of endocervical organoids, e.g. human endocervical organoids a Wnt proficient medium is used, i.e. a medium comprising an activator of Wnt signaling such as Wnt3a and/or RSPO1. In certain embodiments, this medium is essentially free or free from a cAMP signaling activator such as forskolin. For example, a suitable medium may comprise EGF, e.g. in an amount of 1-50 ng/ml such as about 10 ng/ml, fibroblast growth factor 10 (FGF-10), e.g. in an amount of 20-250 ng/ml such as about 100 ng/ml, noggin, e.g. in an amount of 20-250 ng/ml such as about 100 ng/ml, a ROCK inhibitor such as Y-27632, e.g. in an amount of about 10 µM, and an inhibitor or TGF-β signaling, e.g. TGF-β R kinase inhibitor IV. Further, the medium may contain Wnt3a and RSPO1, e.g. from a conditioned medium.

In addition to the above-mentioned components, the cell culture medium of the organoid culture may also comprise a suitable 3D cell culture matrix supporting the growth of organoids such as a basement membrane - like matrix, e.g. a Matrigel®, BME® or EHS® matrix. By embedding in a suitable matrix such as Matrigel® and providing a suitable composition of niche factors in the medium, stem cells from the primary isolate will expand and differentiate into compact structure, i.e. epithelial organoids, which can grow indefinitely.

Stem cells present in squamous or columnar cervical organoids were found to be potential cells of origin for cervical squamous cell carcinoma or cervical adenocarcinoma. Thus, the organoids of the present invention can be used as models for improving diagnosis and/or therapy of cervical disorders, particularly cervical disorders involving pathological cell growth including malignant or benign cell growth, more particularly selected from cervical cancer, precancerous conditions, cervical neoplastic lesions or cervical metaplasias. In certain embodiments, the cervical disorders may be cervical squamous cell carcinoma or precursor conditions thereof such as precancerous conditions, neoplastic lesions or metaplasias resulting from cervical squamous cells. In certain embodiments, the cervical disorders may be cervical adenocarcinoma or precursor conditions thereof such as precancerous conditions, neoplastic lesions or metaplasias resulting from cervical glandular cells including adenomas. Since the organoids may be derived from primary human stem cells, they are excellent tools for use in personalized medicine, e.g. for developing therapeutic protocols individually adapted to the stem cell donor.

Still a further aspect of the present invention relates to the use of the cervix epithelial cell organoid culture and/or the biobank for medical applications, and drug screening. For example, the use may comprise determining the efficacy of a therapeutic compound in the treatment of cervical disorders as described above, in particular cervical adenocarcinoma or cervical squamous cell carcinoma, a cervical precancerous condition, a cervical neoplastic lesion or a cervical metaplasia, e.g. for personalized medicine and/or for the identification of a therapeutic compound suitable in the treatment of cervical adenocarcinoma, cervical squamous cell carcinoma, a cervical precancerous condition, a cervical neoplastic lesion or a cervical metaplasia, e.g. for drug screening.

Another example for a medical application is the use of the cervix epithelial cell organoid culture and/or the biobank as a test system for immunotherapy such as cancer immunotherapy, e.g. for testing the efficacy of immune cells, e.g. T cells in immunotherapy, e.g. the testing of immune cells, e.g. T cells obtained from a patient to be treated or obtained from a heterologous source, e.g. by determining antigen-specific immune responses or for identifying novel neo-antigens and/or neo-epitopes. These neo-antigens or neo-epitopes are useful for cancer vaccine design, particularly for personalized immune interventions.

Still a further aspect of the present invention is based on the finding that the stromal compartment of the cervix provides specific growth factors or inhibitors which influence the outgrowth of specific cell lineages by activating or inhibiting Wnt signaling. Thus, stromal factors such as DKK2, DKK3, Axin2, RSpondin1, RSpondin2 or RSpondin3 are useful targets for the diagnosis and/or therapy of cervical disorders, as described above.

Accordingly, the invention relates to a method of diagnosing a cervical disorder comprising the determination of the presence and/or amount of a stromal factor such as DKK2, DKK3, Axin2, RSpondin1, RSpondin2 or RSpondin3 in a sample from a subject, e.g. in a cervix tissue sample and/or a body fluid sample, and optionally comparing the determined amount with a reference amount which may be derived from a healthy subject or a group of healthy subjects. The method may involve determination of a stromal factor on the protein level by methods known in the art, e.g. by immunologic methods using appropriate reagents, e.g. antibodies or antibody fragments specifically directed against said stromal factor. In further embodiments, the method may involve determination of a stromal factor on the level of nucleic acid molecules, e.g. on the transcript level, by methods known in the art, e.g. by nucleic acid amplification and/or hybridization using appropriate reagents, e.g. primers and/or probes specific for said nucleic acid molecule. This aspect of the invention also relates to a kit comprising at least one reagent for the determination of a stromal factor as indicated above and the use of the kit for the diagnosis of a cervical disorder as described above.

Further, the invention, relates to compounds and methods for the therapy of a cervical disorder as described above. This aspect relates to the use of (i) a stromal factor such as DKK2, DKK3, Axin2, RSpondin1, RSpondin2 or RSpondin3 or a nucleic acid molecule encoding said stromal factor, or of (ii) an inhibitor of such a stromal factor or an inhibitor of a nucleic acid molecule coding therefor as an active agent for the modulation of pathological cervical cell growth. The active agent may be administered in a therapeutically effective amount to a subject in need thereof. The therapeutic goal of this administration is the provision of reduction or elimination of the pathological cell growth by modulating, i.e. stimulating or inhibiting, Wnt signaling depending on whether the pathological cervical cell growth is caused by, associated with, or accompanied by pathologically decreased or increased Wnt signaling.

The present invention also relates to a pharmaceutical composition comprising an active agent selected from (i) a stromal factor such as DKK2, DKK3, Axin2, RSpondin1, RSpondin2 or RSpondin3 or a nucleic acid molecule encoding said stromal factor or (ii) an inhibitor of such a stromal factor or an inhibitor of a nucleic acid molecule coding therefor, and pharmaceutically acceptable carrier.

A stromal factor may be administered by means known in the art for the administration of therapeutic polypeptides. In particular embodiments, a recombinant stromal factor is used. A nucleic acid molecule encoding the stromal factor may be administered by means known in the art for the administration of therapeutic nucleic acid molecules. The nucleic acid molecule may be present as such, or located on a viral or non-viral vector in operative linkage with expression control elements allowing expression in the subject to be treated.

Exemplary inhibitors may be selected from antibodies including monoclonal, chimeric, humanized or human antibodies or antigen-binding fragments of such antibodies specifically directed against said stromal factor. An antibody may be administered by means known in the art for the administration of therapeutic antibodies. In particular embodiments, a recombinant stromal factor is administered. Further exemplary inhibitors may be selected from inhibitory nucleic acid molecules including antisense molecules and inhibitory RNA molecules such as siRNA molecules specifically directed against a transcript of said stromal factor. An inhibitory nucleic acid molecule may be administered by means known in the art for the administration of such agents. In particular embodiments, the inhibitory nucleic acid molecule is administered in suitable vesicles, e.g. cationic vesicles such as liposomes, and/or conjugated to a heterologous moiety, e.g. selected from fatty acids, lipids, and saccharides.

Furthermore, the invention relates to the use of vitamin A for the prevention or treatment of cervical disorders, as described above, as well as related pathologies, e.g. pathologies involving pathological cell growth including malignant or benign growth affecting epithelial transition zones in other organs such as the cornea-conjunctiva junction, the esophagogastric junction, the gastro-duodenal junction, the ileocecal junction and the anorectal junction. The term "vitamin A" particularly includes retinol, retinal retinoic acid, retinoic acid esters and vitamin A derivatives, i.e. compounds demonstrating physiological vitamin A activity, including provitamin A compounds, e.g., carotenoids such as beta-carotene.

According to the present invention, vitamin A may be administered orally, e.g. in vitamin A-enriched diet, as a nutritional supplement, and/or as a medicament. The therapeutically effective dose may be determined by the skilled practitioner.

In preferred embodiments, the present invention is defined by the following items:
1. A method for the production of a cervix epithelial cell organoid culture, said method comprising the steps:
   (a) cultivating cervix stem cells in a suitable cell culture medium, under conditions wherein an organoid is formed, and
   (b) optionally obtaining an organoid from the cultivation step (a).
2. The method of item 1, wherein the cervix stem cells are endocervical stem cells, particularly human endocervical stem cells.
3. The method of item 1 or 2, wherein the cell culture medium is a Wnt proficient medium, e.g. a medium comprising Wnt and RSPO1.
4. The method of item 2 or 3, wherein the organoid comprises columnar endocervical epithelial cells.
5. The method of item 4, wherein the columnar endocervical epithelial cells are positive for the cellular marker KRT7 and/or KRT8.
6. The method of item 1 or 2, wherein the cell culture medium is a Wnt deficient medium, e.g. a medium being essentially free of Wnt and RSPO1 .
7. The method of item 6, wherein the cell culture medium contains a cAMP pathway agonist such as forskolin.
8. The method of item 6 or 7, wherein the organoid comprises stratified ectocervical epithelial cells.
9. The method of item 8, wherein the squamous stratified ectocervical epithelial cells are positive for the marker KRT5.
10.The method of item 1 or 2, wherein the cervix stem cells are ectocervical stem cells, particularly human ectocervical stem cells.
11.The method of item 10, wherein the cell culture medium is a Wnt deficient medium, e.g. a medium essentially free of Wnt and RSPO1.
12.The method of item 11, wherein the culture medium contains a cAMP pathway agonist such as forskolin.
13.The method of any one of the items 10 to 12, wherein the organoid comprises squamous stratified ectocervical epithelial cells.
14. The method of item 13, wherein the squamous ectocervical epithelial cells are positive for the marker KRT5.
15.The method of any one of the preceding items, wherein the cell culture medium contains EGF, noggin, FGF-10, N-acetyl-L-cysteine, nicotinamide, a TGF-β R kinase inhibitor IV, and/or a ROCK inhibitor.
16.A cervix epithelial cell organoid culture which can be stably propagated.
17.The organoid culture of item 16, as obtainable by the method of any one of the items 1 to 15.
18.The organoid culture of item 17, which is a squamous stratified ectocervical epithelial cell culture.
19.The organoid culture of item 18, wherein the squamous ectocervical epithelial cells are positive for the marker KRT5.
20.The organoid culture of claim 17, which is a columnar endocervical epithelial cell culture.
21.The organoid culture of item 20, wherein the columnar endocervical epithelium cells are positive for the marker KRT7 and/or KRT8.
22.A biobank, comprising a plurality of different organoid cultures of any one of the items 16 to 21.
23. Use of an organoid culture of any one of the items 16 to 21, or a biobank of item 22, for the testing of a medicament or an immune cell in the treatment of a cervical disorder, particularly a cervical disorder involving abnormal cell growth including malignant or benign cell growth, more particularly selected from cervical cancer such as cervical adenocarcinoma or cervical squamous cell carcinoma.
24.Use of an organoid culture of item 18 or 19, for the testing of a medicament or an immune cell in the treatment of cervical squamous cell carcinoma or a precancerous condition, neoplastic lesion or metaplasia resulting from cervical squamous cells.
25. Use of an organoid culture of item 20 or 21, for the testing of a medicament or an immune cell in the treatment of cervical adenocarcinoma or a precancerous condition, neoplastic lesion or metaplasia resulting from cervical glandular cells.
26.A screening method for the identification of a compound suitable in the treatment of a cervical disorder selected from cervical squamous cell carcinoma, or a precancerous condition, neoplastic lesion or metaplasia resulting from cervical squamous cells comprising the steps
   (a) providing at least one organoid culture of item 18 or 19,
   (b) contacting at least one compound with the at least one organoid culture provided in step (a), said at least one compound being selected from candidate active agents presumed to be suitable for the treatment of said disorder,
   (c) determining growth or/and propagation of the at least one organoid culture after being contacted with the at least one compound, and
   (d) selecting at least one compound which inhibits growth or/and propagation of at least one organoid culture, as determined in step (c), the selected at least one compound being suitable in the treatment of said disorder.
27.A screening method for the identification of a compound suitable in the treatment of a disorder selected from cervical adenocarcinoma, or a precancerous condition, neoplastic lesion or metaplasia resulting from cervical glandular cells, comprising the steps
   (a) providing at least one organoid culture of item 20 or 21,
   (b) contacting at least one compound with the at least one organoid culture provided in step (a), said at least one compound being selected from candidate active agents presumed to be suitable for the treatment of said disorder,
   (c) determining growth or/and propagation of the at least one organoid culture after being contacted with the at least one compound, and
   (d) selecting at least one compound which inhibits growth or/and propagation of at least one organoid culture, as determined in step (c), the selected at least one compound being suitable in the treatment of said disorder.
28. A method for the diagnosis of a cervical disorder comprising determining the presence and/or amount of a stromal factor in a sample from a subject and optionally comparing the determined amount with a reference amount.
29. The method of item 28 wherein the stromal factor is selected from DKK2, DKK3, Axin2, RSpondin1, RSpondin2 and RSpondin3.
30. A kit for the diagnosis of a cervical disorder comprising a reagent for the determination of a stromal factor.
31.The kit of item 30 wherein the stromal factor is selected from DKK2, DKK3, Axin2, RSpondin1, RSpondin2 and RSpondin3.
32.An active agent selected from (i) a stromal factor or a nucleic acid molecule encoding said stromal factor, or (ii) an inhibitor of such a stromal factor or an inhibitor of a nucleic acid molecule coding therefor for use in the modulation of pathological cervical cell growth.
33.The active agent of item 32 wherein the stromal factor is selected from DKK2, DKK3, Axin2, RSpondin1, RSpondin2 and RSpondin3.
34.The active agent of item 32 or 33 for use in the prevention or treatment of a cervical disorder.
35.Vitamin A for use in the prevention or treatment of cervical disorders including related pathologies affecting the epithelial transitions in other organs.

Further, the invention shall be explained in more detail by the following Figures and Examples.

### Figure legends

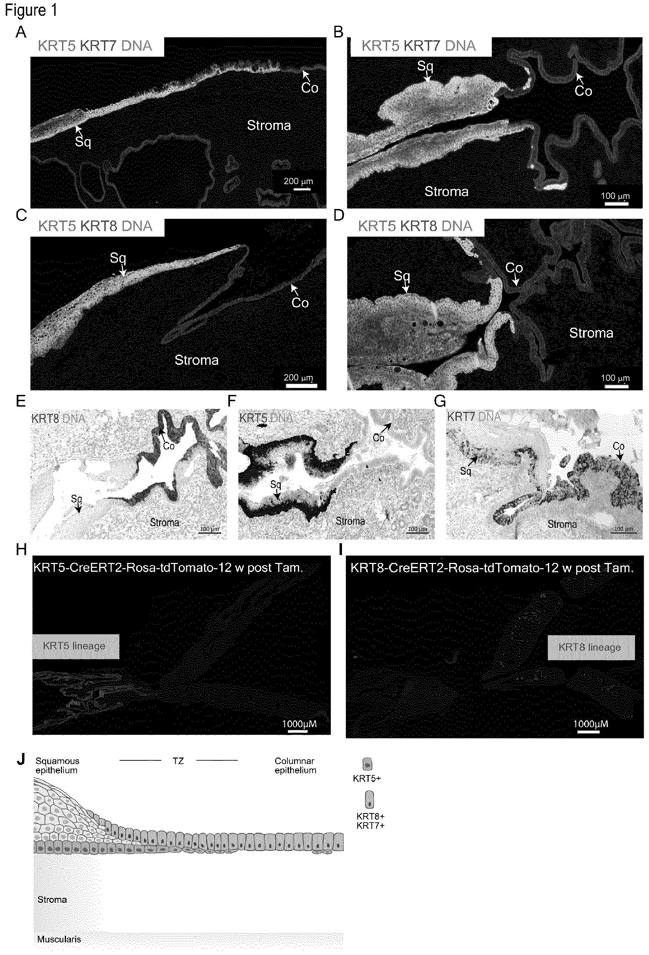

**Figure 1****. The cervix consists of two distinct KRT5+ stratified and KRT7+/8+ columnar epithelial lineages.**
   Transition zone (TZ) including stratified and columnar epithelium from human (A,C) and mouse (B, D) cervix tissue sections immunolabelled with antibodies against KRT5, KRT7, and KRT8; nuclei are shown in blue. (E-G) Single molecule RNA ISH brightfield images of mouse cervix TZ for KRT8, KRT5 and KRT7; nuclei are shown in blue. (H-I) Tiled images of tissue sections from the genital system of 16 weeks old KRT5CreErt2/Rosa26-tdTomato and KRT8CreErt2/Rosa26-tdTomato mice after tamoxifen induction at the age of 4 weeks. (J) Schematic depiction of the stratified and columnar lineages and the TZ of the cervix. Tiled images were acquired with AxioScan imager and are representative of *n* = 3 biological replicates. Arrows indicate squamous epithelium (Sq) and columnar epithelium (Co).

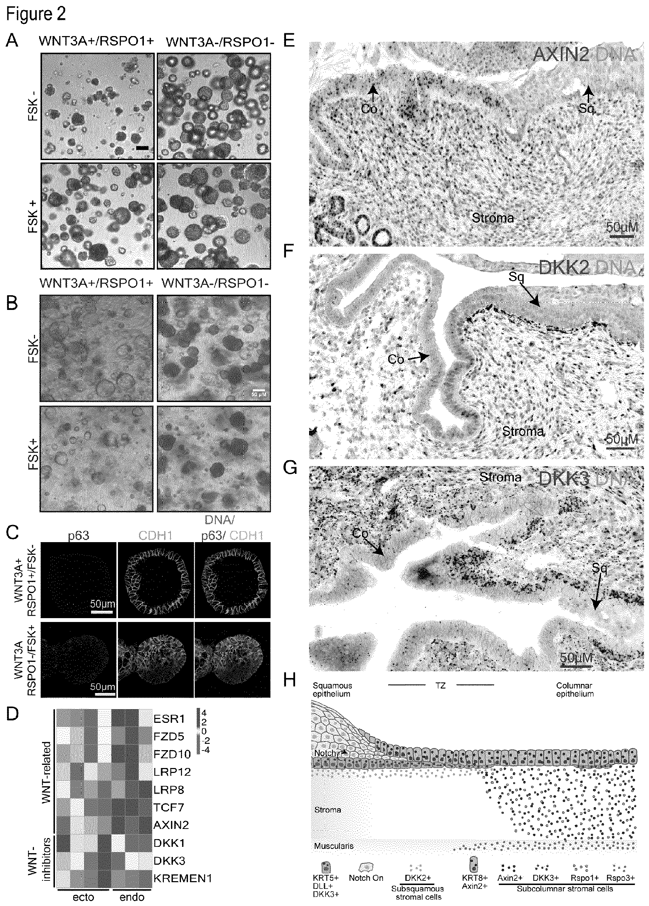

**Figure 2****. Wnt signaling pathway agonists and antagonists play a key role in ecto- and endodcervical development.**
   **(A)** Bright-field images of human ectocervical organoids. Efficient organoid formation depends on absence of Wnt3a and RSPO1 and presence of FSK.
   **(B)** Cells isolated from endocervical tissue grown in Matrigel with different factors. Wnt signaling is essential for columnar organoid formation, while absence of Wnt drives formation of squamous stratified organoids. **(C)** Columnar and stratified organoids derived from endocervix, containing p63⁻ (columnar) and p63⁺ (stratified) cells. Both express the epithelial marker E-cadherin (CDH1). *n* = 5 biological replicates. **(D)** Expression analysis of differentially regulated genes in human ecto- vs endocervical organoids. Wnt-related genes are expressed at higher levels in endocervical, Wnt inhibitors at higher levels in ectocervical organoids. Columns = biological replicates. **(E-G)** Single molecule RNA ISH of mouse TZ for **(E)** AXIN2, **(F)** DKK2, **(G)** DKK3, nuclei are shown in blue. Tiled images were acquired with AxioScan imager and a representative of *n* = 3 biological replicates. (H) A schematic representation of the distinct epithelial lineages and the underlying tissue microenvironment at the TZ. Arrows indicate squamous epithelium (Sq) and columnar epithelium (Co).

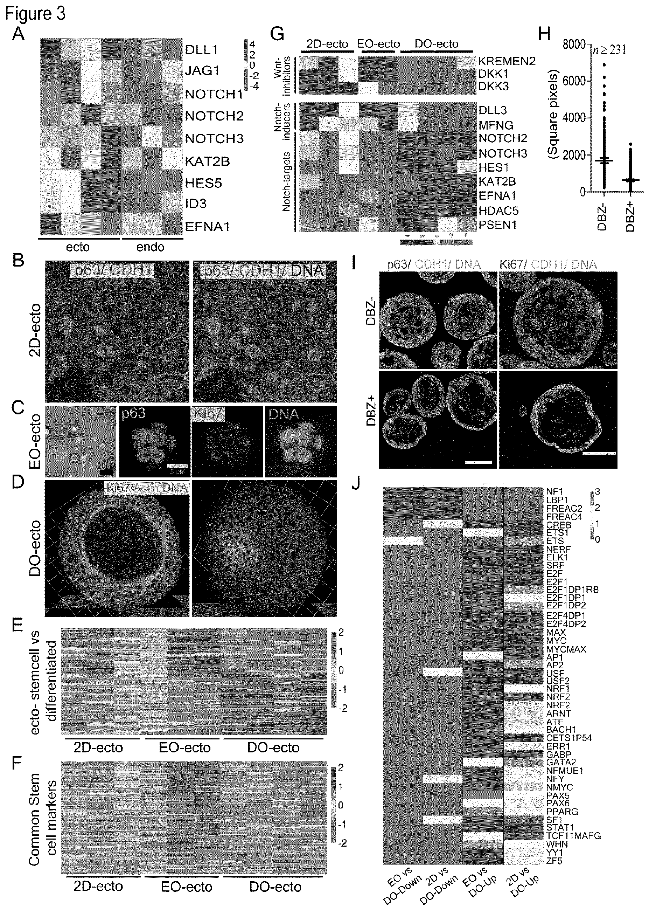

**Figure 3****. Stemness and differentiation of ectocervix depend on Wnt antagonist, Notch and EGFR signaling.**
   (**A**) Expression analysis of differentially regulated genes in human ecto- vs endocervical organoids. Notch-related genes are expressed at higher levels in ectocervix. Columns = biological replicates. (**B**) Confocal images of 2D human ectocervical stem cell cultures immunolabelled for progenitor cell marker p63 and epithelial cadherin (CDH1). (**C, D**) 3D reconstruction of whole-mount confocal images of 3 day-old early ectocervical organoids labelled for p63 and Ki67 (**C**) and a two-week-old differentiated ectocervical organoid labelled for Ki67 and actin (phalloidin) (**D**)**.** (**E, F**) Heatmaps of differentially regulated genes in 2D as well as early and corresponding differentiated ectovervical organoid cultures (**E**) and genes frequently upregulated in stem cells (**F**) (details see Methods section). (**G**) Heatmap of selected differentially expressed genes showing increased Wnt inhibitors and Notch inducers in 2D cultures and early organoids from ectocervix, in contrast to Notch activation-associated genes in differentiated organoids; columns = biological replicates.(**H**) Quantification of the area of human ectocervical organoids grown in the presence or absence of Y-secretase inhibitor (DBZ), *n* = number of organoids, representative of 3 biological replicates; error bars: mean ± s.e.m. (**I**) Confocal images of human ectocervical organoids immunolabelled for CDH1, Ki67 or p63. Inhibition of Notch activation by DBZ prevents differentiation and reduces proliferation. *n* = 3 biological replicates. (**J**) Heatmap showing GSEA enrichment -log10(p-value) of GSEA revealing enrichment of genes upregulated in stem cells regulated by transcription factors downstream of Notch, EGFR-RAS-MAPK target genes signaling among genes upregulated in 2D and EO ectocervical organoids, while the RAS antagonistic NF1 pathway is enriched among genes highly expressed in differentiated ectocervical organoids.

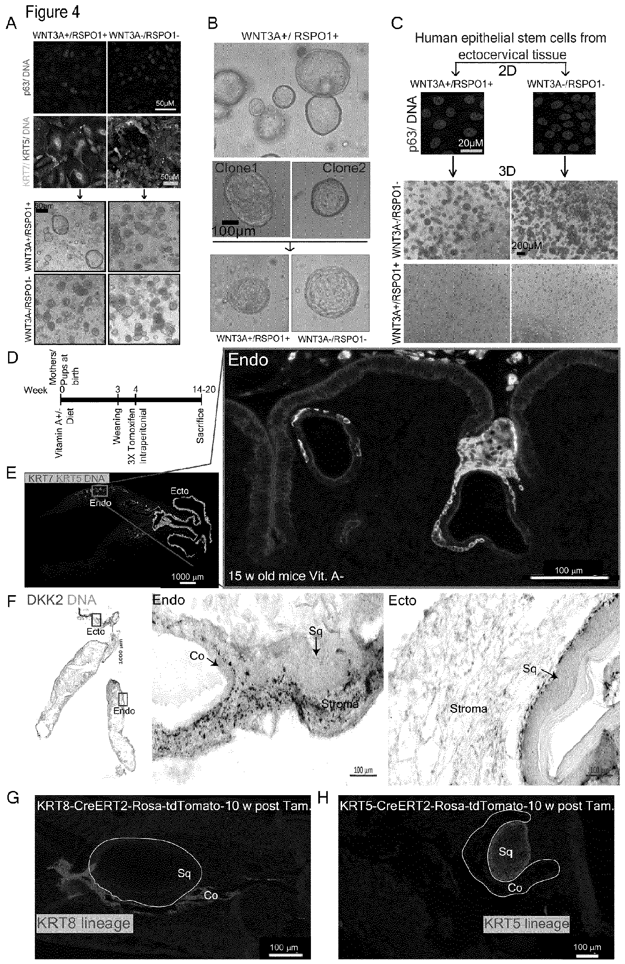

**Figure 4****. Two distinct stem cells from the endocervix give rise to columnar or squamous stratified lineages depending on the microenvironment.**
   (**A**) Wnt deficient medium enriches for p63⁺/KRT5⁺ endocervical stem cells that only give rise to stratified organoids, while Wnt proficient medium supports both KRT7⁺ and p63⁺/KRT5⁺ cells, which can give rise to columnar or stratified organoids, depending on culture conditions (**B**) Endocervical stem cells that give rise to columnar epithelium are unipotent and fail to transdifferentiate into stratified organoids. Single endocervical organoids were grown in Wnt proficient medium, dissociated into single cells and transferred to Wnt proficient or deficient medium. (**C**) Confocal images of ectocervical epithelial cells grown in 2D. p63⁺ cells are present in Wnt proficient and Wnt deficient media but organoids are formed only in Wnt deficient medium. (**D**) Treatment scheme of Vitamin A deficient diet study of WT and lineage tracing mouse. (**E**) Tissue sections from genital system of C57BL6 mouse fed a vitamin A deficient diet for 15 weeks were labelled with antibodies against KRT7 and KRT5. Zoom: outgrowth of subcolumnar KRT5+ stem cells that give rise to squamous metaplastic epithelium in the endocervix. (**F**) Single molecule RNA ISH of tissue from a mouse fed with a vitamin A deficient diet. Expression of DKK2 is enhanced in endocervical stroma. Boxed areas in panel **F** are magnified on right. (**G, H**) Lineage tracing in KRT8CreErt2/Rosa26-tdTomato (**G**) and KRT5CreErt2/Rosa26-tdTomato (**H**) mice fed a vitamin A deficient diet reveals that squamous metaplasia arising in the endocervix is negative for KRT8-tdTomato (**G**) and positive for KRT5-TdTomato (**H**) lineage markers. Fluorescent and brightfield tiled images were acquired with AxioScan imager. Data representative of *n* = 3 biological replicates.

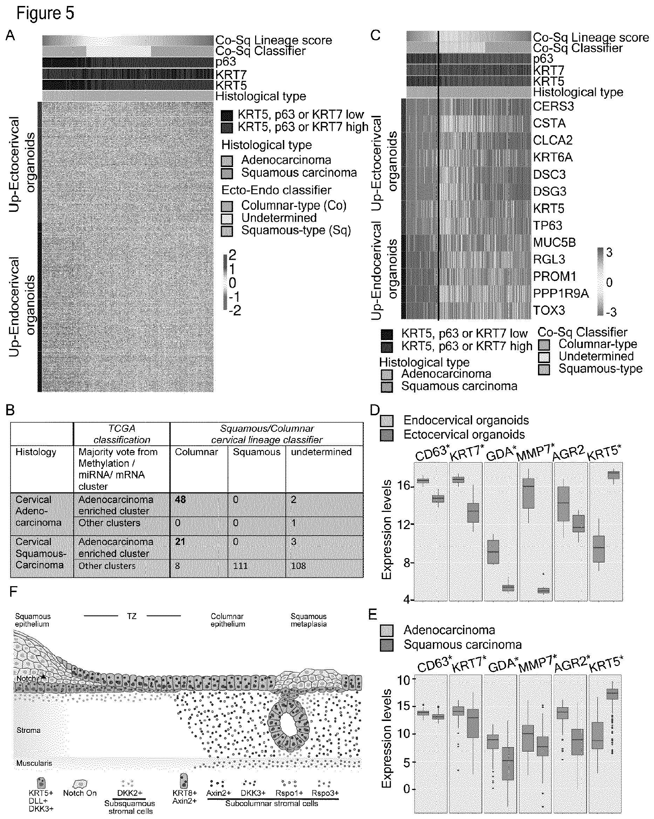

**Figure 5****. Cervical squamous carcinomas originate from KRT5⁺ and adenocarcinomas from KRT7⁺ stem cells.**
   (**A**) Expression profiles of SCC and ADC correlate well with genes differentially expressed between ecto- and endocervical organoids. (**B**) Classification of cancer samples based on majority voting from hierarchical mRNA and miRNA or methylation status clustering suggests that 29 samples are histologically incorrectly diagnosed as squamous carcinoma. (**C**) Heatmap showing the mean-substracted expression for selected bimodal genes in cancer samples that are differentially expressed in squamous and columnar organoids. Color denotes fold-change from mean gene expression across all samples. (**D**, **E**) Expression profiles of proposed squamo-columnar junction markers together with KRT5 in 302 cervical cancer samples (**D**) and in cervical organoids (**E**). Expression of these markers is higher in endocervical organoids (n=6) and ADCs (n=51) compared to ectocervical organoids (n=10) and SCCs (n=251), in contrast to KRT5 expression; * = p<0.05. (**F**) Model depicting the KRT5⁺ and KRT7⁺ stem cell organization and Wnt/Notch microenvironment in TZ and during squamous metaplasia.

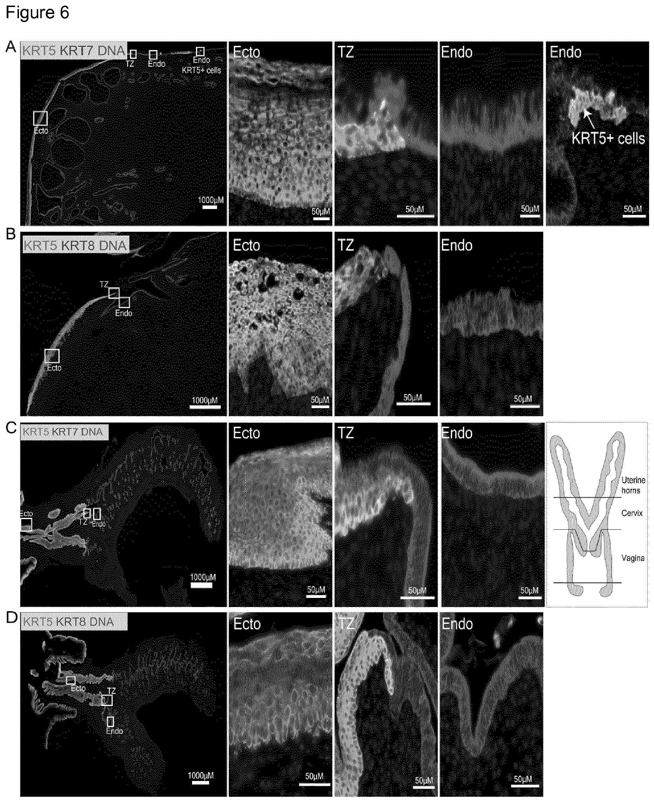

**Figure 6**. **Cervix consists of KRT5+ stratified and KRT7+/KRT8+ columnar epithelium.**
   Human (**A**, **B**) and mouse (**C**, **D**) cervix tissue sections including stratified and columnar epithelium as well as the TZ were labelled with antibodies against KRT5, KRT7 and KRT8; nuclei are shown in blue. Boxed areas are magnified on the right. Images are representative of n=3 biological replicates.
**Figure 7****. Expression of keratins in ecto- and endocervix.**
   Tiled brightfield images of sections showing entire mouse genital system labelled with single molecule RNA-ISH for KRT5 (**A**), KRT8 (**B**) and KRT7 (**C**); nuclei are shown in blue. Boxes are magnified on the right. Images are representative of n=3 biological replicates.
**Figure 8****. Culture conditions for human and mouse ectocervical organoids derived from single epithelial stem cells.**
   Bright-field images showing, (**A**) two-week old human ectocervical organoids grown in the absence of indicated growth factors. NIC - nicotinamide, NAC - N-acetylcysteine. (**B**) Time course of organoids grown from single ectocervical stem cells. (**C**) Mouse ectocervical organoid formation quantified by area; red line: 70 µm diameter. (**D**) Effect of growth factors on ectocervical organoid size. Red line: organoids with > 50 µm diameter, corresponding to > 2000 square pixels; n = number of organoids. (**E**) Maintenance of stemness from P1 to P17 in mouse ectocervical organoids. (**F**, **G**) Confocal images of sections from human and murine cervix and organoids show identical morphology with basal progenitor cells (p63) and stratified epithelium **(E-**cadherin-CDH1) (**F**); only basal cells proliferate (Ki67) (**G**); nuclei: blue, scale bars: 50 µm. (**H-I**) Confocal images of ecto- and endocervical organoids immunolabelled with antibodies against KRT5 and KRT7 reveal their expression is restricted to ectocervical (**H**) and endocervical (**I**) organoids, respectively; nuclei are shown in blue. (**J**) Analysis of differential expression in ecto- vs endocervical organoids point to a distinct expression profile of cytokeratins. Data representative of n = 3 biological replicates.

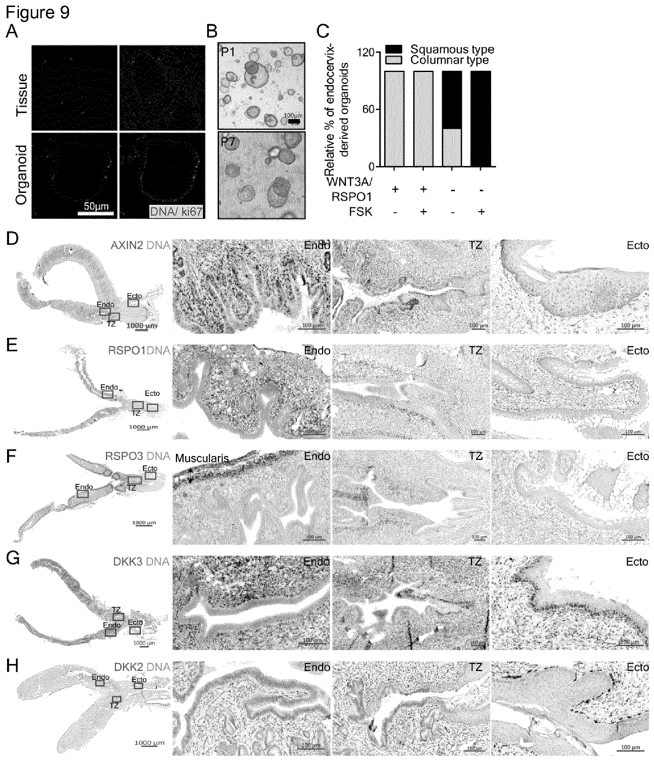

**Figure 9****. Wnt microenvironment controls growth of endocervical organoids.**
   (**A**) Confocal images showing similar distribution of Ki67 in human endocervical tissue and organoids. (**B**) Bright-field images of human endocervical organoids at passage 1 and 7; scale bar: 100 µm. (**C**) Percentage of columnar and squamous stratified organoids formed from human endocervical stem cells in the presence of different growth factors. **(D-H)** Tiled brightfield images of entire mouse genital system sections labelled with single molecule RNA-ISH for AXIN2 (**D**), RSPO1 (**E**), RSPO3 (**F**), DKK3 (**G**), DKK2 (**H**); nuclei are shown in blue. Boxed areas are magnified at right. Data representative of n=3 biological replicates.

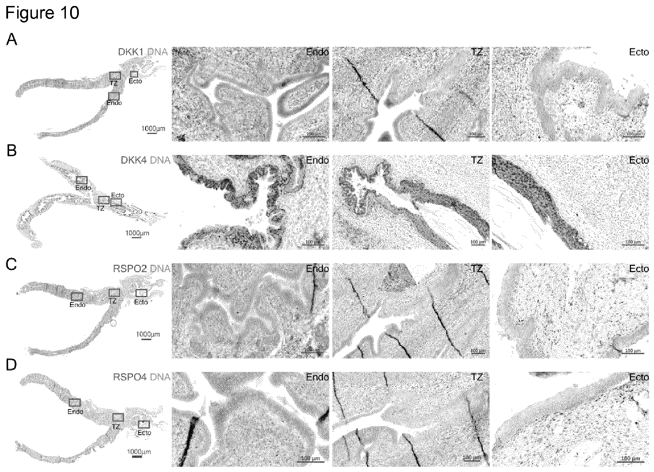

**Figure 10****. Microenvironmental signaling molecules in the cervix.**
   Tiled brightfield images of entire mouse genital system sections labelled with single molecule RNA-ISH for DKK1 (**A**), DKK4 (**B**), RSPO2 (**C**), RSPO4 (**D**); nuclei are shown in blue. Boxed areas are magnified at right. Data representative of n=3 biological replicates.

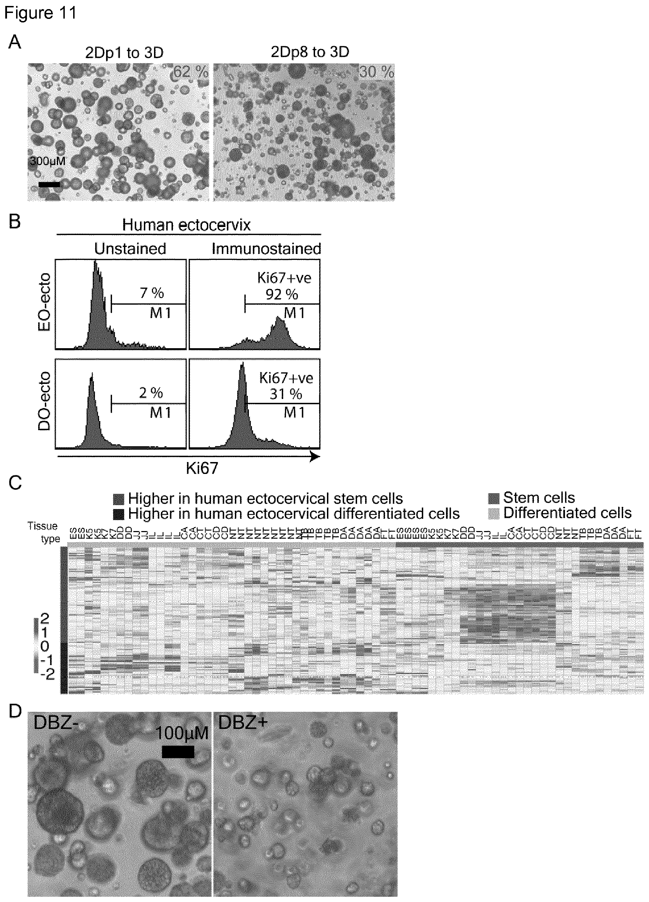

**Figure 11****. Ectocervical stemness and differentiation.**
   (A) Bright-field images of organoids derived at passage 1 or 8 from 2D-ectocervical stem cells. Percentages indicate organoid forming ability. (**B**) Percentage of Ki67+ proliferating cells in EO-ecto and DO-ecto organoid-derived cells. (**C**) Heatmap of genes concordantly up- or downregulated both in ectocervical stem cells vs differentiated cells and in a similar comparisons from the ground state stem cell data set (see methods section for details). Expression levels in stem cells and their corresponding differentiated cells from 13 different tissue types from the ground state stem cell data set are shown. DA-Distal Airway; NT-Nasotubular epithelium ; TB-Tracheobronchial epithelium; FT-Fallopian tube; CA-Colon ascendens, CD-Colon descendens, CT-Colon transversum, DD-Duodenum, ES-Esophagus, IL-Ileum, JJ-Jejunum, K5-Keratin 5+ esophageal cells, K7-Keratin 7+ esophageal cells. (**D**) Phase contrast images of human ectocervical organoids in the presence or absence of DBZ. Data representative of n=3 biological replicates.

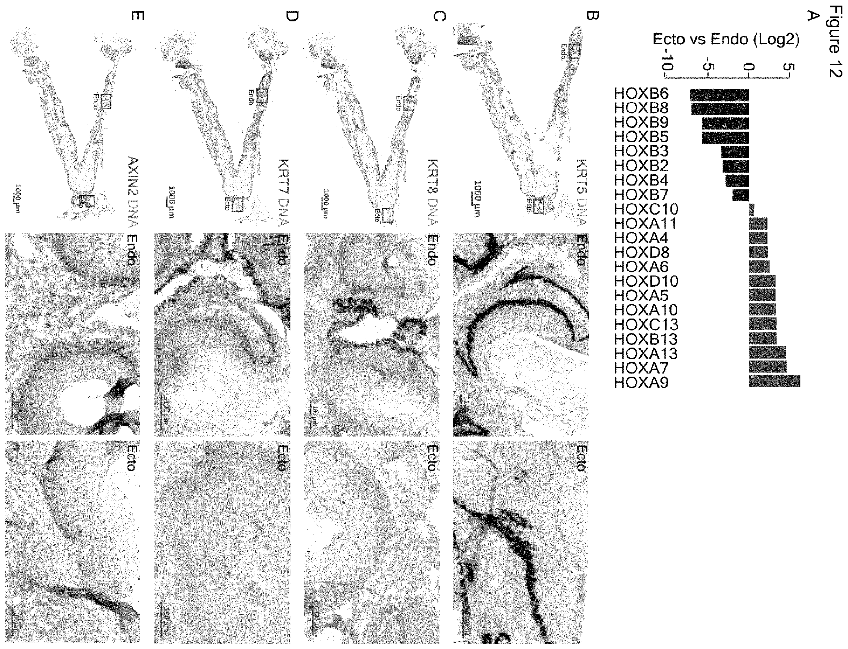

**Figure 12****. Regulation of HOX genes and squamous metaplasia by the tissue microenvironment.**
   (A) Expression analysis of differentially regulated genes in human ecto- vs endocervical organoids reveals a unique set of developmental HOXB genes upregulated in endocervical organoids that is distinct from HOX genes regulated in ectocervix. (**B-E**) Tiled brightfield images of entire genital system sections from mice fed on a Vitamin A deficient diet. Single molecule RNA-ISH labelling for KRT5 (**B**), KRT8 (**C**), KRT7 (**D**), AXIN2 (**E**); nuclei are shown in blue. Boxed areas are magnified on the right. Data representative of n=3 biological replicates.

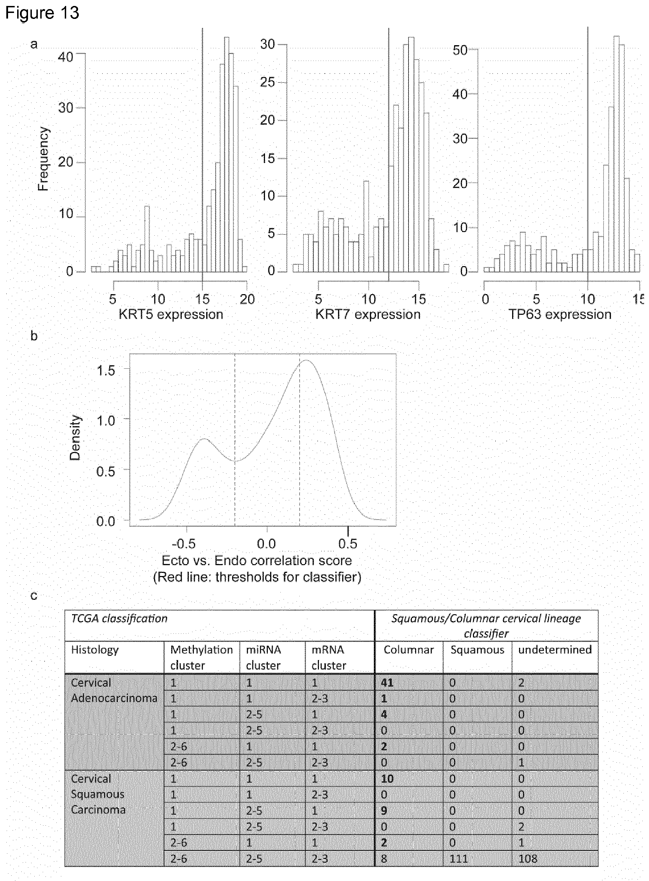

**Figure 13****. Endo- and ectocervical lineage markers as improved cancer classifiers**
   (A) Distribution of expression values and selected thresholds used to define high and low mRNA expressing samples for KRT5, KRT7 and TP63. (**B**) Distribution of Co-Sq lineage Scores used for classification of cancer samples in squamous-like (> 0.2), columnar-like (< -0.2) or undetermined (-0.2 to 0.2) groups. (**C**) Classification of cancer samples according to histopathological diagnosis, TCGA clusters based on genome-wide methylation by NMF, hierarchical clustering based on miRNA expression and global mRNA expression as well as similarity to squamous and columnar lineages as proposed in this work.
**Figure 14****.**
   **Labelling for bimodally expressed proteins in normal cervix, SCC and ADC.** Tissue sections from normal tissue, SCC and ADC of cervix were stained with hematoxylin and eosin or labelled with antibodies against KRT5, KRT7, KRT8, AGR2, GDA, MUC5B and CSTA ; nuclei are shown in blue. Magnifications of the boxed areas are shown in the insets. Data representative of n=5 donors.

### Examples

### 1. Materials and methods

### 1.1 Antibodies and Chemicals

The following antibodies and chemicals were used: mouse-anti-p63 (Abcam, # ab375), rabbit-anti-p63 (Abcam, # ab53039), mouse-anti-E-Cadherin (BD Biosciences, # 610181), rabbit-anti-Ki67 (Abcam, # ab16667), mouse/rat-anti-Ki67-FITC (eBioscience, # 11-5698), mouse-anti-KRT5 (Sigma, # C-7785), rabbit-anti-KRT5 (Abcam, # ab52635), rabbit-anti-cytokeratin 5-Alexa488 (Abcam, # ab193894), mouse-anti-KRT7 (Santa Cruz, # sc-23876), rabbit-anti-cytokeratin 7 (Abcam, # ab181598), rabbit-anti-cytokeratin 7-Alexa555 (Abcam, # ab209601), rabbit-anti-CSTA (Cystatin A) (Sigma # HPA001031), rabbit-anti-AGR2 (Proteintech, # 12275-1-AP), mouse-anti-MUC5B (Abcam, # ab77995), rabbit-anti-GDA (Sigma # HPA019352), Hoechst (Sigma, #B2261), Draq5 (Cell Signaling, #4085), Y-secretase inhibitor XX (DBZ) (Calbiochem # 565789) and p38 inhibitor SB202190 (Sigma, # S7067). Secondary antibodies labelled with the fluorochromes Cy2, Cy3 or Cy5 were obtained from Jackson ImmunoResearch Laboratories.

### 1.2 Mouse experiments

All procedures involving animals were approved by the national legal as well as institutional and local authorities at the Max Planck Institute for Infection Biology. Wild-type C56BL6, KRT5CreErt2 (43) and KRT8CreErt2 (44) mice were obtained from the Jackson Laboratory. These strains were bred to Rosa-tdTomato (45) mice in order to generate mice expressing a fluorophore in Cre-expressing cells. For lineage analysis for the cell of origin of Krt5+ or KRT8+ cells, Cre recombinase was induced in female mice by administering tamoxifen (Sigma) intraperitoneally at 0.25 mg g⁻¹ body weight in 50 µl corn oil at week 4 on three consecutive days. Mice were euthanized at 14-20 weeks and the genital tracts removed for further analysis.

### 1.3 Depletion of retinoid signaling in mice using Vitamin A-deficient diet

At birth experimental mice and their mothers were placed on a vitamin A-deficient test diet (SAFE, U8978P-0074) or control diet with added vitamin A at physiological levels of 6 IU/g (SAFE, U8978P-0075) following a protocol developed for BALB/c mice (25). Littermates were weaned at week 3 of age and maintained on the deficient or control diet for a period of 14-20 weeks before being sacrificed for further analysis.

### 1.4 Mouse cervical medium

Cervical cells were cultured in ADF medium (Invitrogen, # 12634) supplemented with 12 mM HEPES (Invitrogen, # 15630-056), 1% GlutaMax (Invitrogen, # 35050-038), 1% B27 (Invitrogen, # 17504-044), 1% N2 (Invitrogen, # 17502048), 50 ng/ml mouse epidermal growth factor (EGF) (Invitrogen, # PMG8043), 100 ng/ml mouse noggin (Peprotech, # 250-38-100), 100 ng/ml human fibroblast growth factor (FGF)-10 (Peprotech, # 100-26-25), 1.25 mM N-acetyl-L-cysteine (Sigma, # A9165-5G), 10 mM nicotinamide, (Sigma, # N0636), 2 µM TGF-β R Kinase Inhibitor IV (Calbiochem, # 616454), 10 µM ROCK inhibitor (Y-27632) (Sigma, # Y0503), 1% penicillin/streptomycin (Gibco, # 15140-122) with or without 25% Wnt3A- and 25% R-spondin1-conditioned medium, as described in Willert et al (46) and Farin et al (47).

### 1.5 Human ectocervical (Wnt deficient) medium

The medium consisted of ADF, 12 mM HEPES and 1% GlutaMax, supplemented with 1% B27, 1% N2, 0.5 µg/ml hydrocortisone (Sigma, # H0888-1G), 10 ng/ml human EGF (Invitrogen, # PHG0311), 100 ng/ml human noggin (Peprotech; # 120-10C), 100 ng/ml human FGF-10 (Peprotech, # 100-26-25), 1.25 mM N-acetyl-L-cysteine, 10 mM nicotinamide, 2 µM TGF-β R kinase Inhibitor IV, 10 µM ROCK inhibitor (Y-27632), 10 µM forskolin (Sigma, F6886) and 1% penicillin/streptomycin.

### 1.6 Human endocervical (Wnt proficient) medium

The medium consisted of ADF, 12 mM HEPES, 1% GlutaMax, supplemented with 1% B27, 1% N2, 10 ng/ml human EGF, 100 ng/ml human noggin, 100 ng/ml human FGF-10, 1.25 mM N-acetyl-L-cysteine, 10 mM nicotinamide, 2 mM TGF-β R Kinase Inhibitor IV and 10 µM ROCK inhibitor (Y-27632) with 25% Wnt3A- and 25% R-spondin1 conditioned medium.

### 1.7 Epithelial stem cell isolation from human and mouse cervix

Human ecto- and endocervix samples were provided by the Department of Gynecology, Charité University Hospital, Berlin, Germany. Scientific usage of the samples was approved by the ethics committee of the Charité University Hospital, Berlin (EA1/059/15); informed consent to use their tissue for scientific research was obtained from all subjects. Only anatomically normal tissues were used, within 2-3 h after of removal. Mouse cervix was removed from euthanized 4-8 week old healthy female wild type BALB/c mice (from Charles River) immediately preceding the isolation of the cells. Tissue samples were washed thoroughly in sterile PBS (Gibco, # 14190-094) and minced with surgical scissors. Minced tissue was incubated in 0.5 mg/ml collagenase type II (Calbiochem, # 234155) for 2.5 h at 37°C in a shaker incubator. Tissue and dissociated cells were pelleted by centrifugation (5 min at 1000 g, 4°C), supernatant discarded, cells resuspended in TrypLE express (Gibco, # 12604021) and incubated for 15 min at 37°C in a shaker incubator. After dissociation the cell and tissue pellet was resuspended in ADF (Invitrogen) medium and passed through a 40 µm cell strainer (BD Falc, # 352340) to separate the single dissociated cells from tissue pieces. Cells were pelleted by centrifugation (5 min at 1000xg, 40°C), resuspended in either human ecto- or endocervical or mouse cervical medium and cultured either directly as organoids or in 2D.

### 1.8 Human epithelial stem cell culture and maintenance in 2D

Human epithelial stem cells isolated from the tissue were resuspended in either ecto- or endo cervical medium and plated in collagen-coated tissue culture flasks. Cells were incubated at 37°C, 5% CO₂ in a humidified incubator. Once they reached 70-80% confluence, cells were detached using TrypLE Express, and centrifuged at 1000xg for 5 min at 40°C. The cells were then used for culturing organoids or for maintenance of 2D stem cells. 2D stem cells were maintained by seeding the 2D cells from P1 into tissue culture flasks containing lethally irradiated J2-3T3 fibroblast feeder cells in ecto- or endocervical medium. Medium was replaced and irradiated fibroblasts added every 4 days until the colonies reached a confluence of 60-70%, at which stage they were detached and reseeded onto freshly irradiated feeders at a 1:5 ratio or cryopreserved for later use.

### 1.9 Organoid culture and maintenance

Cells isolated from tissue or the stem cells grown in 2D culture were mixed with 50 µl of ice cold Matrigel (BD, # 356231) at a density of 20,000 cells, and the Matrigel droplet was placed in a pre-warmed 24-well plate and allowed to polymerize for 10 min at 37°C. The Matrigel droplet was then overlaid with 500 µl of pre-warmed human ecto- or endocervical medium. Cultures were kept at 37°C, 5% CO₂ in a humidified incubator for 2-3 weeks and medium replaced every four days. For passaging the organoids, Matrigel was first dissolved by adding 1 ml of ice cold ADF and pipetting up and down 5 times. Organoids were collected in a 15 ml Falcon tube and a further 4 ml of ice cold ADF medium was added and organoids resuspended well to completely dissolve the Matrigel, followed by centrifugation at 300xg for 5 mins at 4°C. Medium was discarded and the ectocervical and mouse organoids were incubated with 1 ml of TrypLE Express for 30 min at 37°C followed by mechanical fragmentation using a fire-polished glass Pasteur pipette by vigorous pipetting (8-10 times) to generate single cells. The single cells were then seeded at a 1:10 ratio back into Matrigel for expanding and culturing. Endocervical organoids were subjected to mechanical fragmentation as described above after centrifugation to generate fragments that were seeded back into Matrigel at 1:5 ratio. Matrigel was allowed to polymerize for 10 min at 37°C, overlaid with pre-warmed medium and cultured as described above.

### 1.10 Organoid forming ability

Stem cells were counted and a defined number resuspended in 50 µl of Matrigel to generate organoids as described above. Between 2-3 weeks after plating images were taken of the whole well and the number and area of organoids formed were determined using ImageJ to calculate organoid forming efficiency.

### 1.11 Immunofluorescent histochemistry

Organoids were washed with cold PBS five times to remove Matrigel before fixing with 4% paraformaldehyde for 1 h at room temperature (RT) followed by washing with PBS twice. Organoids were then subjected to dehydration in an ascending ethanol series followed by isopropanol and acetone for 20 min each. The dehydrated organoids were paraffin embedded and 5 µm sections cut on a Microm HM 315 microtome. Mouse and human tissues were extensively washed with PBS and fixed using 4% PFA overnight at RT. Samples were subjected to dehydration in an ascending ethanol series followed by isopropanol and xylene (60 mins each) followed by paraffinization using a Leica TP1020 tissue processor. The tissue was embedded and 5 µm sections cut on a microtome. For immunostaining, paraffin sections were deparaffinized and rehydrated, followed by treatment with antigen retrieval solution (Dako, # S1699). Sections were blocked using blocking buffer (1% BSA and 2% FCS in PBS) for 1 h at RT. Primary antibodies were diluted in blocking buffer and incubated for 90 min at RT followed by five PSB washes before 1 h incubation with secondary antibodies diluted in blocking buffer along with Hoechst or Draq5. Sections were washed with PBS five times and mounted using Mowiol. Images were acquired with a Leica TCS SP8 confocal microscope.

Fresh epithelial isolates were grown on collagen-coated coverslips in 2D and fixed with 4% paraformaldehyde for 30 min at RT. Cells were permeabilized and blocked with 0.5% Triton X-100 and 1% BSA in PBS. Primary antibodies were diluted in 1% BSA in PBS and incubated for 1 h at RT followed by three washes in PSBT (0.1% Tween 20 in PBS), followed by 1 h incubation with secondary antibodies diluted in 1% BSA in PBS along with Hoechst or Draq5. Coverslips were washed three times with PBST and once with PBS and mounted using Mowiol. Images were acquired on a Leica TCS SP8 confocal microscope. Images were processed with Adobe Photoshop; 3D reconstruction was done with the Volocity 6.3 software package (Perkin Elmer).

### 1.12 Whole mount staining

Matrigel was removed from the organoids by extensive washing with ice cold PBS prior to fixation (4x 45 min) and allowed to settle by gravity to maintain the 3D structure. Organoids were then fixed using pre-warmed (37 °C) 3.7% PFA for 1 h at RT followed by three PBST washes. Permeabilization and blocking was performed overnight at 40 °C using 5% donkey serum, 1% FCS, 0.05% Tween20, 2% Triton X-100, 0.02% sodium azide in PBS. Organoids were incubated with primary antibodies diluted in blocking buffer (5% donkey serum, 1% FCS, 0.25% Triton X-100, 0.02% sodium azide in PBS) at 4°C for 3-5 days followed by three PBST washes for 45 min each at RT. Next, organoids were incubated with secondary antibodies diluted in blocking buffer for two days at 4°C followed by one PBST wash for 45 min and three washes with PBS containing 5% glycerol for 45 min each. Organoids were then carefully transferred to an ibidi µ-slide (# 81822) together with some PBS and glycerol solution and Z stack images were acquired with a confocal microscope and image processing and 3D reconstructions were done using Volocity 6.3 software.

### 1.13 Single-molecule RNA in situ hybridization (RNA-ISH).

For single molecule RNA in situ labelling, paraffin embedded 10 µm tissue sections were used with RNAscope 2.5 HD Red Reagent kit (Advanced Cell Diagnostics). Hybridizations were performed according to the manufacture's protocol. In each experiment, positive (PPIB) and negative (DapB) control probes were used as per the manufacturer's guidelines. Tiled bright field images were obtained with Axio Scan.Z1 tissue imager (Zeiss). Images were further processed with Zen 2.3 (Blue edition) image analysis software and further compiled using Adobe illustrator.

### 1.14 RNA isolation and quality control

Microarrays were hybridized for human ectocervical cells cultured in 2D in Wnt-deficient medium (n=3 biological replicates from 2 human donors) or as organoids (EO: n=3 biological replicates from 3 human donors, DO: n=4 biological replicates for 4 human donors), human endocervical cells cultured in 2D Wnt-proficient medium (n=3 biological replicates from 3 human donors) or as DO organoids (n=3 biological replicates from 3 human donors), as well as mouse cervical EO and DO organoids cultured in Wnt-proficient or - deficient medium, respectively (n=2 biological replicates per condition). In the absence of any pre-existing knowledge on expected effect sizes sample sizes were selected based on available samples. Cells and organoids were pelleted and resuspended in 1 ml Trizol (Life Technologies) and RNA was isolated according to the manufacturer's protocol. Quantity of RNA was measured using a NanoDrop 1000 UV-Vis spectrophotometer (Kisker) and quality was assessed by Agilent 2100 Bioanalyzer with an RNA Nano 6000 microfluidics kit (Agilent Technologies).

### 1.15 Microarray expression profiling and Data analysis

Microarray experiments were performed as single-color hybridizations on custom whole genome human 8x60k Agilent arrays (Design ID 048908) and Agilent Feature Extraction software was used to obtain probe intensities. The extracted single-color raw data files were background corrected, quantile normalized and further analyzed for differential gene expression using R (48) and the associated BioConductor package LIMMA (49) (Supplementary Information Table 2). Microarray gene expression comparisons between groups were performed using unpaired tests for all human comparisons. R was also used for all statistical analyses unless stated otherwise. Mann-Whitney-U test was used for comparisons of gene expression in SCJ marker genes with a threshold of p<0.05. Microarray data have been deposited in the Gene Expression Omnibus (GEO; www.ncbi.nlm.nih.gov/geo/) of the National Center for Biotechnology Information and can be accessed with the GEO accession number GSE87076. These data form part of the present disclosure.

The signature of differentially expressed genes between ectocervical 2D/EO vs DO organoids was selected from all genes with a false discovery rate (FDR) < 0.05 and log2 fold change < -1.5 or > 1.5 in any of the two comparisons (2D vs. DO or EO vs. DO) and the largest absolute fold change from both comparisons and possible replicate probes was taken for each gene.

### 1.16 Analysis of stem cell related genes

Raw data from different microarray data sets obtained from adult tissue stem cells (SC) cultured on feeder cells and corresponding differentiated cells from air liquid interface (ALI), Matrigel or self-assembly sphere (SAS) were downloaded from GEO (GSE57584, GSE66115, GSE69453, GSE65013, GSE32606, GSE69429, GSE49292) and normalized together using method 'RMA-sketch' with Affymetrix Power Tools. We assessed differentially expressed genes between SC and corresponding differentiated cell cultures for normal esophagus, Barrett's esophagus, gastric cardia, duodenum, jejunum, ileum, colon ascendens, colon transversum, colon descendens, KRT5+ and KRT7+ fetal esophageal cells, fallopian tube, nasal turbinated epithelium, tracheobronchial epithelium and distal airway epithelium. We selected stem cell-related genes as those genes with significant (adjusted p-value < 0.05) up- or down-regulation (abs(logFC) > 1) in at least 5 out of 18 comparisons.

### 1.17 Gene Set Enrichment Analysis (GSEA)

We performed a pre-ranked GSEA analysis using GSEA software v2.1.0 (50, 51) obtained from http://software.broadinstitute.org/gsea. The t-statistics from comparisons of ectocervical organoids (2D vs. Differentiated organoids or Early organoids vs. Differentiated organoids) were used to rank probes and enrichment of MSigDB Motif gene sets [http://software.broadinstitute.org/gsea/msigdb] (c3.all.v5.1.symbols.gmt) was computed using standard settings, collapsing probe sets within genes using the Max_probe method and using 1000 permutations. For further analysis we kept only motif gene sets that were significant in at least one of the up or down regulated genes in the two comparisons mentioned above at FDR < 5%. For the heatmap visualization, we chose the smallest p-value for motif gene sets referring to the same transcription factor use the negative Iog10 of this value for visualization.

### 1.18 Cervical cancer data

Expression data (Level 3 processed RNASeq_v2) was obtained for 302 unique samples with available histological diagnosis from The Cancer Genome Atlas (TCGA) data portal (https://gdc-portal.nci.nih.gov/). This data was generated within the Cervical Squamous Cell Carcinoma and Endocervical Adenocarcinoma project (TCGA-CESC) and is a superset of the published cohort (52). Per gene expression levels were extracted from "*.rsem.genes.normalized_results" files using custom scripts. Public clinical sample annotations for those samples were also obtained from the same source. Aggregated features including clustering results based on DNA methylation, mRNA and microRNA expression was obtained from the Cervical and Endocervical Cancer (CESC) project Firehose site of TCGA (53). For the details on the majority vote see Fig. 13. To classify samples into squamous-like and columnar-like classes, the gene expression levels were log2 transformed and Z-score was applied to make genes comparable. A squamous vs columnar organoid signature was defined based on the fold changes between ectocervical squamous and endocervical columnar differentiated organoids for 2,834 genes with FDR < 0.05 and absolute log2 fold change > 1, selecting the probe with lowest p-value for each gene. Spearman correlation coefficients (referred to as Co-Sq Score) were computed between Z-scored gene expression values from each cancer sample and the corresponding fold change for the same gene from the squamous vs columnar organoid signature. We defined samples with Co-Sq Score > 0.2 as squamous-like, those with < -0.2 as columnar like and all other as 'undetermined'. Applying the same procedure to 1,000 random sets of genes of the same size with the same fold changes produced sample correlation coefficients generally lower than |0.06|. Thresholds for classification of samples into KRT5-high/low and KRT7-high/low as well as TP63 high/low classes were selected manually to separate the highest cluster from all other samples (Fig. 13). For simplicity reasons we combined all diagnoses with an adenoma component (Endocervical Adenocarcinoma, Endometrioid Adenocarcinoma, Mucinous Adenocarcinoma and Adenosquamous Carcinoma) into Cervical Adenocarcinoma.

### 2. Results

### 2.1 Distinct cellular origins of squamous and columnar epithelium

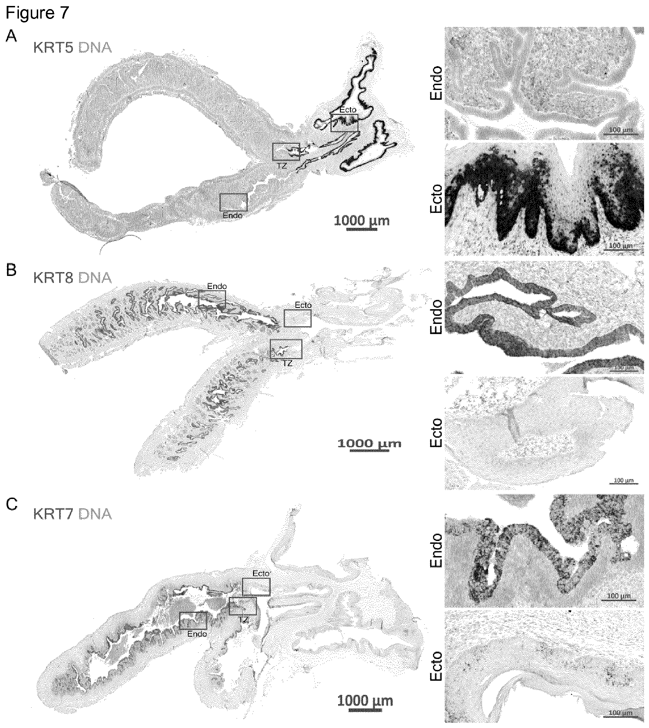

To obtain deeper insight into the cellular composition and molecular determinants of transition zone (TZ) maintenance, we carried out a detailed analysis of marker profiles in human and mouse cervical epithelium. Strikingly, our comprehensive, unbiased analysis including the entire endo- and ectocervix regions failed to detect any specific cell type-restricted exclusively to the TZ in contrast to the prevailing concept. Instead, we observed two distinct epithelial lineages, with KRT5 expressed throughout the squamous stratified epithelium and KRT7/KRT8 expressed throughout the columnar epithelium. At the TZ there is an overlap of both lineages, where KRT5+ basal cells appear to displace overlying KRT7+/KRT8+ columnar cells to form squamous stratified epithelium (Fig. 1A-D and Fig. 6A-D). RNA-ISH confirmed that KRT8 and KRT5 expression was restricted to the columnar epithelium and basal/parabasal cells of squamous epithelium, respectively (Fig. 1 E-F, and Fig. 7A-B). In contrast to previous reports describing a discrete KRT7 population, restricted to the TZ (4, 5), we observed KRT7 expression at high levels throughout the endocervical epithelium and sparse expression in the ectocervical epithelium (Fig. 1G and Fig. 7 C). We also observed that patches of subcolumnar KRT5+ cells occurred sporadically beneath KRT7+/KRT8+ cells within the endocervix (Fig. 6A). These islands of KRT5+ cells may correlate with foci of squamous metaplasia that are frequently observed within the endocervix and account for 10% of premalignant squamous intraepithelial lesions (SIL) (11, 12).

Further, using KRT5CreErt2/Rosa26-tdTomato and KRT8CreErt2/Rosa26-tdTomato mice for genetic lineage tracing we confirmed the presence of two distinct epithelial lineages in the cervix (Fig. 1 H-I and J). In these mice, Cre was induced by tamoxifen injection 4 weeks after birth. At 16 weeks of age, KRT5+ cells exclusively populated the stratified epithelium, including all differentiated cells, while KRT8+ cells exclusively generated endocervical epithelium.

### 2.2 Wnt agonists and antagonists in epithelia and stroma orchestrate TZ

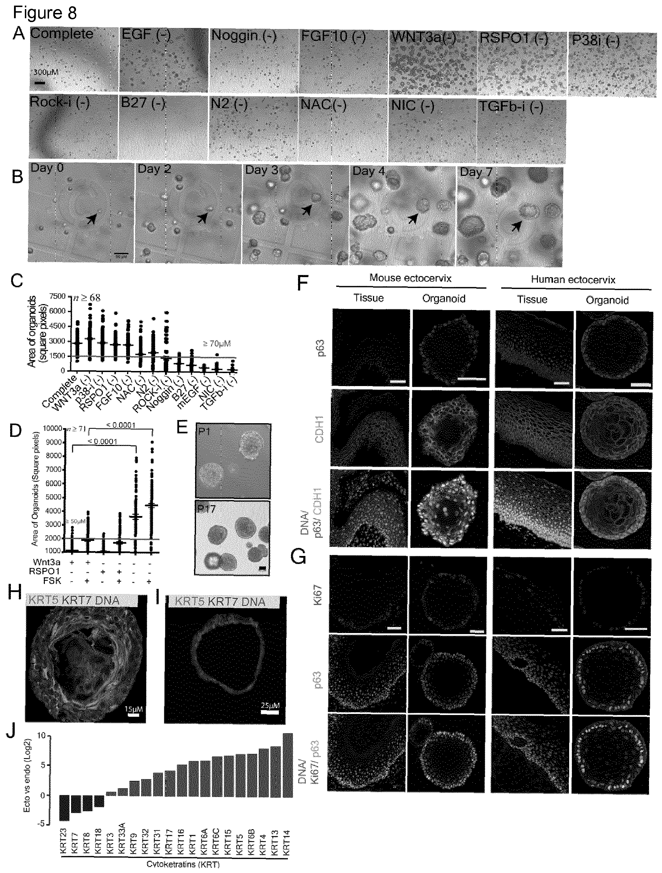

To gain insight into the factors that regulate the two lineages and maintain the TZ, we established and defined conditions that facilitate long-term in vitro propagation of ecto- and endocervical epithelial stem cells as 3D organoids. Wnt signaling was described to be essential for generation and long-term maintenance of adult epithelial stem cell-derived organoids from various tissues so far described as shown by the requirement of Wnt3a and R-spondin 1 in the tissue-specific organoid culture media (13). In contrast to this, the presence of Wnt3a and RSpondin 1 (RSPO1) in the culture media was found to be detrimental for the formation and expansion of human and mouse squamous stratified organoids derived from single ectocervical stem cells (Fig. 2A and Fig. 8A-C). While, the presence of epidermal growth factor (EGF), fibroblast growth factor 10 (FGF-10), and the inhibition of transforming growth factor beta (TGF-β) and bone morphogenetic protein (BMP) signaling are essential for long-term maintenance of these organoids. Squamous stratified organoid growth was further increased in the presence of the cAMP pathway agonist forskolin (FSK) (Fig. 2A and Fig. 8D-E). These organoids are KRT5+/KRT7- and fully recapitulate the in vivo tissue architecture, with stratified epithelial layers decorated with the adhesion molecule E-cadherin (CDH1) (Fig. 8F and H). The outer layer consists of p63+ basal cells, which differentiate into parabasal cells with p63 staining fading out towards the lumen. Also, typical of ectocervical tissue, only basal cells express the proliferation marker Ki67 (Fig. 8G).

In contrast to the ectocervix, stem cells derived from both proximal and distal endocervix give rise to organoids consisting of a simple columnar epithelial layer when cultured in the presence of Wnt proficient medium containing Wnt3a and RSPO1 (Fig. 2B-C). These organoids faithfully resemble the in vivo endocervical epithelium, are KRT7+/KRT5- and exhibit sporadic Ki67 staining (Fig.9A and 8I. Their self-renewal capacity in culture can be maintained for more than seven months (Fig. 9B). Further, transcriptional profiling of organoids derived from human ecto- and endocervix revealed distinct keratin expression patterns (Fig. 8J).

Strikingly, if cells derived from endocervix tissue were cultured in Wnt deficient medium (FSK+ medium without Wnt3a or RSPO1), they gave rise to p63+ stratified organoids, resembling those derived from ectocervix (Fig. 2B-C, 9C). Since the formation of columnar rather than squamous organoids from endocervical stem cells was dependent on supplementation with Wnt agonists, we investigated the source of Wnt signaling in the cervix. Microarray analysis of organoids and RNA-ISH showed that the transcriptional regulation of Wnt in the endocervix diverges from that in the ectocervix: Wnt agonists are upregulated in columnar epithelium, while the Wnt antagonists Dickkopf WNT signaling pathway inhibitor 3 (DKK3), DKK1 and KREMEN1 are upregulated in squamous epithelium (Fig. 2D, E, and G).

Further, we observed that the spatial distribution of extrinsic Wnt agonists and antagonists in the underlying stroma defines the borders between the two epithelial types. Both the Wnt agonist RSPO1 and its downstream target Axin2 are highly expressed in the lamina propria (stroma) beneath the columnar epithelium and RSPO3 in the muscularis of the endocervix (Fig. 2E, 9D-F). Notably, the Wnt antagonist DKK2 is specifically expressed in stroma proximal to the basal cells of the ectocervical squamous epithelium, which express high levels of DKK3 (Fig. 2F-H, 9G-H). In contrast to the ectocervix, high levels of DKK3 expression were observed in the endocervical stroma, while expression of DKK1 was negligible in either region of the cervix (Fig. 10A). Expression levels of DKK4, RSPO2 and RSPO4 also did not show notable regional variation (Fig. 10B-D). Thus, the epithelium of the cervix is maintained by two distinct stem cell populations whose fate is determined by opposing Wnt signaling microenvironments, which are established through the interplay of the epithelial and stromal compartments of the endo- and ectocervix respectively, with a defined switch at the TZ.

### 2.3 Wnt antagonists, Notch and EGFR signaling maintain ectocervical stemness and differentiation

Next, we sought to identify the cellular pathways that control self-renewal and differentiation in human ectocervical tissue. Microarray analysis showed that squamous ectocervical organoids have a higher expression of Notch-related genes than organoids derived from the endocervical columnar epithelium (Fig 3A). We thus carried out a comparative analysis of 2D cells (2D-ecto), three-day-old early organoids (EO-ecto), and two-week-old, mature differentiated organoids (DO-ecto). 2D cultures were enriched for CDH1+ and p63+ cells, with >60% and >30% of cells showing organoid-forming potential at passage 1 and 8, respectively (Fig. 3B, Fig. 11A). Early organoids consist of 8-16 cells that are undifferentiated and positive for Ki67 and p63 (Fig. 3C and Fig. 11B). Mature organoids consist of several stratified differentiated layers with more than two-thirds of cells differentiated and less than one-third of cells proliferating (Fig. 3D and Fig. 11B). Gene expression patterns of cells from 2D cultures and early organoids show high similarity and display a distinct set of differentially expressed genes compared to mature organoids (Fig. 3E). Recent studies reported that stem cells from diverse tissue types show similar transcriptional signatures compared to the large divergence observed in the ensuing differentiated tissues (14). Comparative analysis of the ectocervical cells (either 2D or EO) and differentiated cell expression profiles to that of frequently upregulated genes in stem cells from diverse tissue types confirmed a high similarity shared with the ectocervical 2D cells and EO in contrast to DO-ecto cells (Fig. 3F). This is further supported by the expression profile of genes that are concordantly up- or downregulated in ectocervical 2D-ecto and EO-ecto vs. DO-ecto cells with those of ground state stem cells derived from different tissue types vs. their respective differentiated cells (Fig. 11C). Thus 2D-ecto and EO-ecto define characteristics of ectocervical stem cells.

A survey of genes that are upregulated in ectocervical stem cells compared to differentiated cells revealed high expression of the Notch ligands Delta-Like Ligand 3 (DLL3) and Manic Fringe (MFNG), the latter facilitating binding of DLL to the Notch receptor (Fig. 3G). In contrast, differentiated cells expressed higher levels of Notch 2 and Notch 3 receptors as well as their targets, including the transcription factor HES1 and Presenilin 1 (PSEN1), a core component of Y-secretase (Fig. 3G). Ectocervical stem cells also showed highly upregulated expression of the WNT antagonists DKK1, DKK3 and the DKK receptor KREMEN2 (Fig. 3G). Concordantly, inhibition of Notch activation using the Y-secretase inhibitor DBZ reduced organoid growth (Fig. 3H, Fig. 11D), as these organoids failed to differentiate and stratify (Fig. 3I). Thus the ectocervical stem cells act as Notch signal-sending cells, while the differentiated cells show the signature of Notch signal-receiving cells, leading to the trans-activating interaction that facilitates differentiation and ultimately epithelial stratification.

Further, gene set enrichment analysis (GSEA) revealed that genes regulated by several transcription factors downstream of Notch ligand and EGF receptor (EGFR)-RAS-MAPK signaling were highly enriched among genes upregulated in ectocervical stem cells, including AP1 (15, 16), CREB, ETS, new ETS-related factor (NERF), ELK1, E2F, SRF, MYC and YY1 (17-20) (Fig. 3J). The two pathways function together to regulate proliferation and differentiation, with the EGFR pathway promoting the expression of Notch DLL ligands (21). On the other hand, genes belonging to the RAS antagonistic NF1 pathway (22) were enriched in genes highly expressed in differentiated cells. Together, these observations indicate that the Wnt antagonists together with EGFR and Notch-inducing pathways regulate ectocervical stemness and differentiation.

### 2.4 The emergence of squamous metaplasia from quiescent KRT5+ stem cells in the endocervix

We performed *in vitro* and *in vivo* analysis to determine the cellular origin and mechanism of squamous metaplasia. Primary endocervix-derived cells showed a clear enrichment of KRT5+ and p63+ cells if cultured in 2D in Wnt deficient medium. After transfer to organoid culture conditions, these cells produced only organoids of the squamous type, even in the presence of Wnt3a /RSPO1 (Fig. 4A). However, if primary endocervix-derived cells were grown in 2D in a Wnt proficient medium such cultures contained only a few KRT5+ or p63+ cells and gave rise to columnar organoids in the presence of Wnt. Yet, the absence of Wnt favored the growth of squamous organoids, including the characteristic basal and parabasal p63+ cells (Fig. 4A). Importantly though, endocervical organoids derived from single cells remained columnar even when transferred to Wnt deficient medium, thus excluding the possibility that columnar cells transdifferentiate to the squamous lineage (Fig. 4B). In contrast, primary ectocervical cells grown in 2D with either Wnt proficient or deficient medium give rise only to stratified organoids in Wnt deficient medium (Fig. 4C).

Although the expression of HOX genes, a family of decisive regulators during embryonic development, is largely unknown for the cervix, HOXA11 has previously been associated with cervix development (23) and deregulation of HOXB2, HOXB4, and HOXB13 have been implicated in cervical carcinogenesis (24). Here we analyzed the pattern of HOX gene expression in the ecto- vs. endocervical organoids (Fig. 12A). Strikingly, we observed substantial differences between the two cultures, supporting the notion that the two tissue types represent different biological lineages in the cervix.

To further consolidate the lineage properties of stratified and columnar epithelial cells and spatial changes in the microenvironment, we performed lineage tracing, single cell RNA-ISH and IHC in a mouse model of squamous metaplasia, induced by retinoid depletion (25). These retinoid-depleted mice showed an upregulation of DKK2 gene expression in the stroma of the endocervix and uterine horns (here also referred to as endocervix), and the emergence of subcolumnar quiescent KRT5+ cells that eventually developed into metaplastic squamous stratified epithelium (Fig. 4D-F and 12B, compare to Fig. 2F and 9H). However, production of the Wnt target Axin2, which is normally expressed in the endocervix, remained unaltered in these mice, while KRT8 and KRT7 expression were restricted to the columnar epithelium (Fig. 4E, Fig. 12C-E). Further, by performing lineage tracing analysis in retinoid depleted KRT5CreErt2/Rosa26-tdTomato and KRT8CreErt2/Rosa26-tdTomato mice, we confirmed that KRT8+ cells give rise to columnar epithelium while KRT5+ cells give rise to squamous metaplasia in the endocervix (Fig. 4G-H). Together, these data demonstrate that the endocervix harbors two distinct, unipotent stem cell populations with the potential to develop columnar or stratified lineages, respectively. Which one is activated thus appears to depend on the microenvironment and the opposing Wnt-related signals in particular. While Wnt agonists support the formation of columnar epithelium, the local upregulation of Wnt antagonist in the stroma drives the proliferation of quiescent KRT5+ reserve cells to cause squamous metaplasia.

### 2.5 Cellular origins of cervical squamous and adenocarcinomas

A number of studies have shown that adult stem cells are susceptible to transformation and often constitute the cells of origin for a variety of cancers (26). The origin of cervical adenocarcinoma (ADC) and squamous cell carcinoma (SCC) is controversial and uncertain. Here we assessed the expression signatures of squamous and columnar cervical organoids to determine the cells of origin of cervical cancers. We retrieved publically available mRNA expression data for 302 cervical cancers from The Cancer Genome Atlas (TCGA, http://cancergenome.nih.gov/). We used the similarity of differential gene expression profiles between ectocervical squamous and endocervical columnar organoids to those between cancer samples to classify the latter into squamous-like, columnar-like or undetermined cases (Fig.5A, Methods). We found that cancers classified as squamous-like matched a histological diagnosis of SCC in all cases (n=111) while for those classified as columnar-like we found 48/77 matching a histological diagnosis of ADC (Fig. 5B). For 111 cases histologically diagnosed as SCC and 3 ADC, we could not determine a clear classification and 29 SCC where assigned to the columnar-like group by our classifier. Importantly, cancer samples classified as columnar-like were mainly KRT5low, KRT7high and p63low, while samples in the squamous-like and undetermined group were mainly KRT5high and p63high with mixed KRT7 status (Fig. 5A), suggesting that the undetermined group could consist of SCCs within or outgrown into columnar endocervix, leading to the presence of contaminating endocervical columnar KRT7+ cells in the samples.

To validate these results, we also obtained clustering results based on genome-wide methylation, global mRNA and microRNA expression data for the same cancer samples from TCGA. The TCGA cluster containing most ADCs in each of the clustering analyses from those three levels of cellular regulation showed strong overlap with our columnar-like class. Using the majority vote among mRNA, miRNA and DNA methylation clusters (Fig. 5B) we find that 69/77 samples from the columnar-like group are also in the TCGA clusters enriched for ADC, while all other TCGA clusters together contain mainly squamous-like and undetermined samples (228/231) (Fig. 5B and S8). Interestingly, 21/29 cancers defined as columnar-like based on our classifier, but histologically classified as SCC, showed strong similarity to ADC according to TCGA molecular profiles and might therefore be misdiagnosed.

A recent study suggested that only a small population of cells located in the TZ (the so-called squamocolumnar junction (SCJ) cells) express KRT7 and that these are the precursors of both SCC and ADC (4). We further investigated the mRNA expression levels in organoids and 302 cervical cancer samples from TCGA with regard to SCJ markers proposed in that study (4), as well as KRT5. In contrast to KRT5, expression of the proposed SCJ markers is significantly higher in healthy endocervical organoids as compared to healthy ectocervical organoids and the same trend is seen in ADC vs. SCC (Fig. 5D-E). This indicates that the reported SCJ cells are not distinct from the endocervical columnar lineage and are not the cells of origin for SCC.

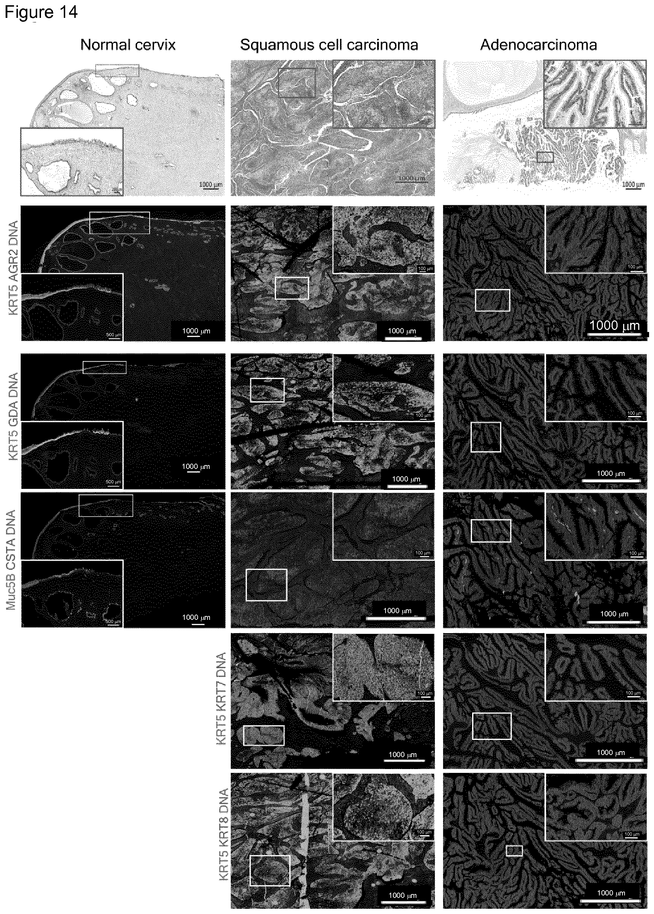

Our study also revealed a set of genes that are differentially expressed between squamous and columnar organoids and show a strong correlation with columnar-like and squamous-like cancers, including MUC5B, KRT5, CSTA, while the proposed SCJ markers KRT7, AGR2 and GDA specifically labelled ADC but not SCC sections (Figure 5C and 14). Thus, the majority of cervical cancers can be divided into two subgroups based on molecular signatures that correlate with signatures of KRT5+ stem cells for squamous or KRT7+/KRT8+ stem cells for columnar cervical epithelia. Together, these results indicate that the cervix harbors two distinct stem cell lineages, reflecting the cells of origin for SCC and ADC, respectively.

### 3. Discussion

The TZs of the mucosal epithelium constitute critical zones of enhanced disposition to infections and carcinogenesis (27-31). Revealing the principles of cellular regulation and homeostasis of these tissue regions is key to understanding the impact of intrinsic and extrinsic disturbances, as well as for prospective and therapeutic disease prevention.

We show that distinct microenvironment conditions and molecular signals from the epithelial and stromal tissues drive the dominance of specific epithelial lineages of the TZ. We reveal Wnt signaling as a key determinant in regulating the homeostasis at borders between two epithelial types. Wnt signaling has been shown to be indispensable for the maintenance and homeostasis of adult stem cells in several mammalian tissues (13, 32). However, here we show that in the cervix, Wnt signaling stimulated by the underlying stroma drives the columnar lineage while imposing quiescence of squamous lineage-specific stem cells that exist in the same milieu. With the transition to a Wnt repressive microenvironment, these quiescent squamous lineage stem cells are activated and replace the columnar epithelia at TZ or as an island of metaplasia within the endocervix. Further, the fact that ADC or SCC arise from two distinct stem cell lineages rather than a common cellular origin has important clinical implications for choice of therapy and suggests that preventive ablation of the SCJ alone may not fully eliminate potential cervical cancer precursor cells (37, 38).

We also found that mice fed with vitamin A deficient diet develop cervical metaplasia throughout the endocervix. Since metaplasia is a pre-neoplastic condition and a major risk factor for carcinogenesis we conclude that with nutritional supplementation with vitamin A we not only can prevent metaplasia but also cancer development.

Our data constitute a major conceptual progress in our understanding of how epithelial junctions are maintained in our body. Accordingly, homeostasis at these sites is not maintained by the transition from one epithelial type to another but rather that the adult tissue is composed of different stem cell populations that are retrieved upon extrinsic signals to generate respective cell lineages, forming the adult tissue. This novel concept on the homeostasis of the mucosal TZs fits well with other recent observations on the mucosal stem cell identity and may stimulate future investigations with therapeutic relevance.

### References

1. Y. S. Fu, Pathology of the Uterine Cervix, Vagina, and Vulva. (Saunders, 2002).
2. A. Singer, The uterine cervix from adolescence to the menopause. Br. J. Obstet. Gynaecol. 82, 81-99 (1975).
3. J. Ferlay et al., GLOBOCAN 2012 v1. 0. Cancer incidence and mortality worldwide: IARC CancerBase, http://globocan.iarc.fr, (28/09/2016). (2013).
4. M. Herfs et al., A novel blueprint for 'top down' differentiation defines the cervical squamocolumnar junction during development, reproductive life, and neoplasia. J. Pathol. 229, 460-468 (2013).
5. M. Jiang et al., Transitional basal cells at the squamous-columnar junction generate Barrett's oesophagus. Nature 550, 529-533 (2017).
6. F. Smedts et al., Keratin expression in cervical cancer. Am. J. Pathol. 141, 497-511 (1992).
7. H. zur Hausen, Papillomaviruses in the causation of human cancers - a brief historical account. Virology 384, 260-265 (2009).
8. D. L. Jacobson, L. Peralta, N. M. Graham, J. Zenilman, Histologic development of cervical ectopy: relationship to reproductive hormones. Sex. Transm. Dis. 27, 252-258 (2000).
9. J. E. Martens et al., Reserve cells in human uterine cervical epithelium are derived from Müllerian epithelium at midgestational age. Int. J. Gynecol. Pathol. 26, 463-468 (2007).
10. O. Reich, S. Regauer, Two major pathways of recurrent high-grade squamous intraepithelial lesions of the cervix. Int. J. Cancer 137, 2520-2521 (2015).
11. F. W. Abdul-Karim, Y. S. Fu, J. W. Reagan, W. B. Wentz, Morphometric study of intraepithelial neoplasia of the uterine cervix. Obstet. Gynecol. 60, 210-214 (1982).
12. E. Burghardt, in Viral etiology of cervical cancer, R. Peto, H. Zur Hausen, Eds. (Cold Spring Harbor Laboratory, 1986).
13. H. Clevers, K. M. Loh, R. Nusse, Stem cell signaling. An integral program for tissue renewal and regeneration: Wnt signaling and stem cell control. Science 346, 1248012 (2014).
14. X. Wang et al., Cloning and variation of ground state intestinal stem cells. Nature 522, 173-178 (2015).
15. P. Ranganathan, K. L. Weaver, A. J. Capobianco, Notch signalling in solid tumours: a little bit of everything but not all the time. Nat. Rev. Cancer 11, 338-351 (2011).
16. M. J. LaVoie, D. J. Selkoe, The Notch ligands, Jagged and Delta, are sequentially processed by alpha-secretase and presenilin/gamma-secretase and release signaling fragments. J. Biol. Chem. 278, 34427-34437 (2003).
17. M. Cargnello, P. P. Roux, Activation and function of the MAPKs and their substrates, the MAPK-activated protein kinases. Microbiol. Mol. Biol. Rev. 75, 50-83 (2011).
18. S. H. Yang, A. D. Sharrocks, A. J. Whitmarsh, MAP kinase signalling cascades and transcriptional regulation. Gene 513, 1-13 (2013).
19. R. Avraham, Y. Yarden, Feedback regulation of EGFR signalling: decision making by early and delayed loops. Nat. Rev. Mol. Cell Biol. 12, 104-117 (2011).
20. M. Li et al., Guanylate binding protein 1 is a novel effector of EGFR-driven invasion in glioblastoma. J. Exp. Med. 208, 2657-2673 (2011).
21. L. Tsuda, R. Nagaraj, S. L. Zipursky, U. Banerjee, An EGFR/Ebi/Sno pathway promotes delta expression by inactivating Su(H)/SMRTER repression during inductive notch signaling. Cell 110, 625-637 (2002).
22. Y. S. Yap et al., The NF1 gene revisited - from bench to bedside. Oncotarget 5, 5873-5892 (2014).
23. H. S. Taylor, G. B. Vanden Heuvel, P. Igarashi, A conserved Hox axis in the mouse and human female reproductive system: late establishment and persistent adult expression of the Hoxa cluster genes. Biol. Reprod. 57, 1338-1345 (1997).
24. R. Lopez et al., HOXB homeobox gene expression in cervical carcinoma. Int. J. Gynecol. Cancer 16, 329-335 (2006).
25. N. Darwiche, G. Celli, L. Sly, F. Lancillotti, L. M. De Luca, Retinoid status controls the appearance of reserve cells and keratin expression in mouse cervical epithelium. Cancer Res. 53, 2287-2299 (1993).
26. A. C. White, W. E. Lowry, Refining the role for adult stem cells as cancer cells of origin. Trends Cell Biol. 25, 11-20 (2015).
27. W. Golusiński, C. R. Leemans, A. Dietz, HPV Infection in Head and Neck Cancer. (Springer, 2016).
28. E. J. Yang et al., Microanatomy of the cervical and anorectal squamocolumnar junctions: a proposed model for anatomical differences in HPV-related cancer risk. Mod. Pathol. 28, 994-1000 (2015).
29. X. Wang et al., Residual embryonic cells as precursors of a Barrett's-like metaplasia. Cell 145, 1023-1035 (2011).
30. S. Rajendra et al., Transcriptionally active human papillomavirus is strongly associated with Barrett's dysplasia and esophageal adenocarcinoma. Am. J. Gastroenterol. 108, 1082-1093 (2013).
31. P. Morbini et al., Markers of squamocolumnar junction cells in normal tonsils and oropharyngeal cancer with and without HPV infection. Histol. Histopathol. 30, 833-839 (2015).
32. T. P. Yamaguchi, Heads or tails: Wnts and anterior-posterior patterning. Curr. Biol. 11, R713-R724 (2001).
33. H. Tian et al., Opposing activities of Notch and Wnt signaling regulate intestinal stem cells and gut homeostasis. Cell Rep 11, 33-42 (2015).
34. R. Nusse, Wnt signaling and stem cell control. Cell Res. 18, 523-527 (2008).
35. J. J. Otero, W. Fu, L. Kan, A. E. Cuadra, J. A. Kessler, Beta-catenin signaling is required for neural differentiation of embryonic stem cells. Development 131, 3545-3557 (2004).
36. M. Paschaki et al., Transcriptomic analysis of murine embryos lacking endogenous retinoic acid signaling. PLoS One 8, e62274 (2013).
37. S. Franceschi, Past and future of prophylactic ablation of the cervical squamocolumnar junction. Ecancermedicalscience 9, 527 (2015).
38. M. Herfs et al., Unique recurrence patterns of cervical intraepithelial neoplasia after excision of the squamocolumnar junction. Int. J. Cancer 136, 1043-1052 (2015).
39. N. Chin, A. B. Platt, G. J. Nuovo, Squamous intraepithelial lesions arising in benign endocervical polyps: a report of 9 cases with correlation to the Pap smears, HPV analysis, and immunoprofile. Int. J. Gynecol. Pathol. 27, 582-590 (2008).
40. N. Kumar et al., Diverse glandular pathologies coexist with high-grade squamous intraepithelial lesion in cyto-histological review of atypical glandular cells on ThinPrep specimens. Cytopathology 20, 351-358 (2009).
41. A. A. Shah, S. K. Jeffus, Z. Zhao, M. H. Stoler, E. B. Stelow, Adjunct p16(INK4a) immunohistochemistry aids the detection of high-grade squamous intraepithelial lesions in endocervical curettage specimens. Am. J. Clin. Pathol. 141, 342-347 (2014).
42. J. Doorbar, Papillomavirus life cycle organization and biomarker selection. Dis. Markers 23, 297-313 (2007).
43. J. R. Rock et al., Basal cells as stem cells of the mouse trachea and human airway epithelium. Proc. Natl. Acad. Sci. U. S. A. 106, 12771-12775 (2009).
44. A. Van Keymeulen et al., Distinct stem cells contribute to mammary gland development and maintenance. Nature 479, 189-193 (2011).
45. L. Madisen et al., A robust and high-throughput Cre reporting and characterization system for the whole mouse brain. Nat. Neurosci. 13, 133-140 (2010).
46. K. Willert et al., Wnt proteins are lipid-modified and can act as stem cell growth factors. Nature 423, 448-452 (2003).
47. H. F. Farin, J. H. Van Es, H. Clevers, Redundant sources of Wnt regulate intestinal stem cells and promote formation of Paneth cells. Gastroenterology 143, 1518-1529.e1517 (2012).
48. R-Core-Team, R: A language and environment for statistical computing. R Foundation for Statistical Computing, Vienna, Austria (2015).
49. M. E. Ritchie et al., limma powers differential expression analyses for RNA-sequencing and microarray studies. Nucleic Acids Res. 43, e47 (2015).
50. V. K. Mootha et al., PGC-1 alpha-responsive genes involved in oxidative phosphorylation are coordinately downregulated in human diabetes. Nat. Genet. 34, 267-273 (2003).
51. A. Subramanian et al., Gene set enrichment analysis: a knowledge-based approach for interpreting genome-wide expression profiles. Proc. Natl. Acad. Sci. U. S. A. 102, 15545-15550 (2005).
52. The Cancer Genome Atlas Research Network, Integrated genomic and molecular characterization of cervical cancer. Nature 543, 378-384 (2017).
53. Broad Institute TCGA Genome Data Analysis Center, Cervical Squamous Cell Carcinoma and Endocervical Adenocarcinoma (Primary solid tumor). Aggregate Analysis Features. Broad Institute of MIT and Harvard. doi:10.7908/C14B30G5, (2015).

## Claims

1. A method for the production of a cervix epithelial cell organoid culture, said method comprising the steps:
(a) cultivating cervix stem cells in a suitable cell culture medium, under conditions wherein an organoid is formed, and
(b) optionally obtaining an organoid from the cultivation step (a).

2. The method of claim 1, wherein the cervix stem cells are endocervical stem cells, e.g. human endocervical stem cells.

3. The method of claim 2, wherein the cell culture medium is a Wnt proficient medium and wherein the organoid comprises columnar endocervical epithelium cells, which are positive for the markers KRT7 and/or KRT8.

4. The method of claim 2, wherein the cell culture medium is a Wnt deficient medium which contains a cAMP pathway agonist such as forskolin and wherein the organoid comprises squamous stratified ectocervical epithelial cells which are positive for the marker KRT5.

5. The method of claim 1, wherein the cervix stem cells are ectocervical stem cells, e.g. human ectocervical stem cells.

6. The method of claim 5, wherein the culture medium is a Wnt deficient medium which contains a cAMP pathway agonist such as forskolin and wherein the organoid comprises squamous stratified ectocervical epithelial cells which are positive for the marker KRT5.

7. A cervix epithelial cell organoid culture which can be stably propagated, particularly an organoid culture, as obtainable by the method of any one of the claims 1 to 6.

8. The organoid culture of claim 7, which is a squamous stratified ectocervical epithelial cell culture.

9. The organoid culture of claim 7, which is a columnar endocervical epithelium cell culture.

10. A biobank, comprising a plurality of different organoid cultures of any one of the claim 7 to 9.

11. Use of an organoid culture of any one of the claims 7 to 9, or a biobank of claim 10, for the testing of a medicament or an immune cell in the treatment of a cervical disorder such as cervical adenocarcinoma, cervical squamous cell carcinoma, a cervical precancerous condition, a cervical neoplastic lesion or a metaplasia.

12. A screening method for the identification of a compound suitable in the treatment of a cervical disorder such as cervical adenocarcinoma, cervical squamous cell carcinoma, a cervical precancerous condition, a cervical neoplastic lesion or a metaplasia comprising the steps
(a) providing at least one organoid culture of any one of claims 7 to 9,
(b) contacting at least one compound with the at least one organoid culture provided in step (a), said at least one compound being selected from candidate active agents presumed to be suitable for the treatment of said disorder,
(c) determining growth or/and propagation of the at least one organoid culture after being contacted with the at least one compound, and
(d) selecting at least one compound which inhibits growth or/and propagation of at least one organoid culture, as determined in step (c), the selected at least one compound being suitable in the treatment of said disorder.

13. A method for the diagnosis of a cervical disorder comprising determining the presence and/or amount of a stromal factor in a sample from a subject and optionally comparing the determined amount with a reference amount, wherein the stromal factor is selected from DKK2, DKK3, Axin2, RSpondin1, RSpondin2 or RSpondin3.

14. A kit for the diagnosis of a cervical disorder comprising a reagent for the determination of a stromal factor selected from DKK2, DKK3, Axin2, RSpondin1, RSpondin2 or RSpondin3.

15. An active agent selected from (i) a stromal factor selected from DKK2, DKK3, Axin2, RSpondin1, RSpondin2 or RSpondin3 or a nucleic acid molecule encoding said stromal factor, or (ii) an inhibitor of such a stromal factor or an inhibitor of a nucleic acid molecule coding therefor for use in the modulation of pathological cervical cell growth.
